# EUROPEAN PATENT APPLICATION

(11) **EP 3 754 025 A1**
(43) Date of publication of application: **23.12.2020**
(21) Application number: 19744448.2
(22) Date of filing: 24.01.2019
(51) Int. Cl.: C12N 15/86, C12N 5/0775, C07K 14/475, A61K 35/28, A61K 38/18

(54) **MESENCHYMAL STEM CELLS EXPRESSING BRAIN-DERIVED NEUROTROPHIC FACTOR AND USE THEREOF**

(30) Priority: 24.01.2018 KR 20180008569
(71) Applicant: Slbigen Inc., Yeonsu-gu Incheon 21983 (KR)
(72) Inventor: SUNG, Young Chul, Seoul 04385 (KR); LEE, Soon Min, Seoul 06762 (KR); KIM, Hey Yon, Seoul 05578 (KR)
(74) Representative: Dehns
(86) International application number: PCT/KR2019/001007
(87) International publication number: WO 2019/147036

(57) **Abstract**

The present invention relates to; a recombinant lentivirus comprising a gene encoding brain-derived neurotrophic factor (BDNF) protein,; and a mesenchymal stem cell transformed with the lentivirus,; and a method for mass-producing a cell therapeutic agent expressing BDNF protein using the same. The mesenchymal stem cell transformed with the recombinant lentivirus of the present invention can regulate the timing of expression of BDNF protein, maintain a high cell proliferation rate and overexpress a BDNF protein, and are excellent in safety. Therefore, a mesenchymal stem cells transfected with the lentivirus may be used for the treatment of various neurological diseases as a stem cell therapeutic agent capable of being mass-produced and maintaining the same effect.

<110>    SLBIGEN Inc.

<120>    MESENCHYMAL STEM CELL EXPRESSING BRAIN-DERIVED NEUROTROPHIC
         FACTOR GENE AND USE THEREOF

<130>    1065041

<150>    KR 10-2018-0008569
<151>    2018-01-24

<160>    16

<170>    KoPatentIn 3.0

<210>    1
<211>    255
<212>    PRT
<213>    Artificial Sequence

<220>
<223>    amino acid sequence of BDNF


<400>    1
Met Phe His Gln Val Arg Arg Val Met Thr Ile Leu Phe Leu Thr Met
  1               5                  10                  15

Val Ile Ser Tyr Phe Gly Cys Met Lys Ala Ala Pro Met Lys Glu Ala
             20                  25                  30

Asn Ile Arg Gly Gln Gly Gly Leu Ala Tyr Pro Gly Val Arg Thr His
         35                  40                  45

Gly Thr Leu Glu Ser Val Asn Gly Pro Lys Ala Gly Ser Arg Gly Leu
     50                  55                  60

Thr Ser Leu Ala Asp Thr Phe Glu His Val Ile Glu Glu Leu Leu Asp
 65                  70                  75                  80

Glu Asp Gln Lys Val Arg Pro Asn Glu Glu Asn Asn Lys Asp Ala Asp
                 85                  90                  95

Leu Tyr Thr Ser Arg Val Met Leu Ser Ser Gln Val Pro Leu Glu Pro
            100                 105                 110

Pro Leu Leu Phe Leu Leu Glu Glu Tyr Lys Asn Tyr Leu Asp Ala Ala
        115                 120                 125

Asn Met Ser Met Arg Val Arg Arg His Ser Asp Pro Ala Arg Arg Gly
    130                 135                 140

Glu Leu Ser Val Cys Asp Ser Ile Ser Glu Trp Val Thr Ala Ala Asp
145                 150                 155                 160

Lys Lys Thr Ala Val Asp Met Ser Gly Gly Thr Val Thr Val Leu Glu
                165                 170                 175

Lys Val Pro Val Ser Lys Gly Gln Leu Lys Gln Tyr Phe Tyr Glu Thr
            180                 185                 190

Lys Cys Asn Pro Met Gly Tyr Thr Lys Glu Gly Cys Arg Gly Ile Asp
        195                 200                 205

Lys Arg His Trp Asn Ser Gln Cys Arg Thr Thr Gln Ser Tyr Val Arg
    210                 215                 220

Ala Leu Thr Met Asp Ser Lys Lys Arg Ile Gly Trp Arg Phe Ile Arg
225                 230                 235                 240

Ile Asp Thr Ser Cys Val Cys Thr Leu Thr Ile Lys Arg Gly Arg
                245                 250                 255


<210>    2
<211>    768
<212>    DNA
<213>    Artificial Sequence

<220>
<223>    nucleotide sequence of BDNF


<400>    2
atgttccacc aggtgagaag agtgatgacc atcctgttcc tgaccatggt gatcagctac      60

ttcggctgca tgaaggctgc ccctatgaag gaggccaaca tcagaggaca gggcggcctg     120

gcctaccccg gcgtgagaac ccacggcacc ctggagagcg tgaacggccc caaggccggc     180

agcagaggcc tgaccagcct ggccgacacc ttcgagcacg tgatcgagga gctgctggac     240

gaggaccaga aggtgagacc caacgaggag aacaacaagg acgccgacct gtacaccagc     300

agagtgatgc tgagcagcca ggtgcccctg gagcctcccc tgctgttcct gctggaggag     360

tacaagaact acctggacgc cgccaacatg agcatgagag tgagaagaca cagcgacccc     420

gccagaagag gcgagctgag cgtgtgcgac agcatcagcg agtgggtgac cgccgccgac     480

aagaagaccg ccgtggacat gagcggcggc accgtgaccg tgctggagaa ggtgcccgtg     540

agcaagggcc agctgaagca gtacttctac gagaccaagt gcaaccccat gggctacacc     600

aaggagggct gcagaggcat cgacaagaga cactggaaca gccagtgcag aaccacacag     660

agctacgtga gagccctgac catggacagc aagaagagaa tcggctggag attcatcaga     720

atcgacacca gctgcgtgtg caccctgacc atcaagagag gcagataa                  768


<210>    3
<211>    376
<212>    PRT
<213>    Artificial Sequence

<220>
<223>    amino acid sequence of TK


<400>    3
Met Ala Ser Tyr Pro Cys His Gln His Ala Ser Ala Phe Asp Gln Ala
  1               5                  10                  15

Ala Arg Ser Arg Gly His Ser Asn Arg Arg Thr Ala Leu Arg Pro Arg
             20                  25                  30

Arg Gln Gln Glu Ala Thr Glu Val Arg Leu Glu Gln Lys Met Pro Thr
            35                  40                  45

Leu Leu Arg Val Tyr Ile Asp Gly Pro His Gly Met Gly Lys Thr Thr
    50                  55                  60

Thr Thr Gln Leu Leu Val Ala Leu Gly Ser Arg Asp Asp Ile Val Tyr
65                  70                  75                  80

Val Pro Glu Pro Met Thr Tyr Trp Gln Val Leu Gly Ala Ser Glu Thr
                85                  90                  95

Ile Ala Asn Ile Tyr Thr Thr Gln His Arg Leu Asp Gln Gly Glu Ile
            100                 105                 110

Ser Ala Gly Asp Ala Ala Val Val Met Thr Ser Ala Gln Ile Thr Met
        115                 120                 125

Gly Met Pro Tyr Ala Val Thr Asp Ala Val Leu Ala Pro His Val Gly
    130                 135                 140

Gly Glu Ala Gly Ser Ser His Ala Pro Pro Pro Ala Leu Thr Leu Ile
145                 150                 155                 160

Phe Asp Arg His Pro Ile Ala Ala Leu Leu Cys Tyr Pro Ala Ala Arg
                165                 170                 175

Tyr Leu Met Gly Ser Met Thr Pro Gln Ala Val Leu Ala Phe Val Ala
            180                 185                 190

Leu Ile Pro Pro Thr Leu Pro Gly Thr Asn Ile Val Leu Gly Ala Leu
        195                 200                 205

Pro Glu Asp Arg His Ile Asp Arg Leu Ala Lys Arg Gln Arg Pro Gly
    210                 215                 220

Glu Arg Leu Asp Leu Ala Met Leu Ala Ala Ile Arg Arg Val Tyr Gly
225                 230                 235                 240

Leu Leu Ala Asn Thr Val Arg Tyr Leu Gln Gly Gly Gly Ser Trp Trp
                245                 250                 255

Glu Asp Trp Gly Gln Leu Ser Gly Thr Ala Val Pro Pro Gln Gly Ala
            260                 265                 270

Glu Pro Gln Ser Asn Ala Gly Pro Arg Pro His Ile Gly Asp Thr Leu
        275                 280                 285

Phe Thr Leu Phe Arg Ala Pro Glu Leu Leu Ala Pro Asn Gly Asp Leu
    290                 295                 300

Tyr Asn Val Phe Ala Trp Ala Leu Asp Val Leu Ala Lys Arg Leu Arg
305                 310                 315                 320

Pro Met His Val Phe Ile Leu Asp Tyr Asp Gln Ser Pro Ala Gly Cys
                325                 330                 335

Arg Asp Ala Leu Leu Gln Leu Thr Ser Gly Met Val Gln Thr His Val
            340                 345                 350

Thr Thr Pro Gly Ser Ile Pro Thr Ile Cys Asp Leu Ala Arg Thr Phe
        355                 360                 365

Ala Arg Glu Met Gly Glu Ala Asn
    370                 375

<210>    4
<211>    1131
<212>    DNA
<213>    Artificial Sequence

<220>
<223>    nucleotide sequence of TK

<400>    4
atggccagct acccctgtca ccagcacgcc agcgccttcg accaggccgc tagaagcaga          60

ggccacagca acagaagaac cgccctgaga cccagaagac agcaggaggc cacagaggtg          120

agactggagc agaagatgcc caccctgctg agagtgtaca tcgatggacc ccacggcatg          180

ggcaagacca caacaaccca gctgctggtg gccctgggca gcagagacga catcgtgtac          240

gtgcccgagc ccatgaccta ctggcaggtg ctgggagcca gcgagaccat cgccaacatc          300

tacaccacac agcacagact ggaccagggc gagatcagcg ccggcgacgc tgccgtggtg          360

atgaccagcg cccagatcac aatgggcatg ccctacgccg tgaccgatgc cgtgctggct          420

ccccacgtgg gcggagaggc cggcagcagc cacgcccctc cccctgccct gaccctgatc          480

ttcgacagac accccatcgc cgccctgctg tgctaccccg ccgctagata cctgatgggc          540

agcatgacac cccaggccgt gctggccttc gtggccctga tcccccctac cctgcccggc          600

accaacatcg tgctgggcgc cctgcccgag gacagacaca tcgacagact ggctaagaga          660

cagagacccg gcgagagact ggacctggcc atgctggccg ccatcagaag agtgtacggc          720

ctgctggcca acaccgtgag atacctgcag ggaggcggca gctggtggga ggactggggc          780

cagctgagcg gcaccgccgt gcctccccag ggcgccgagc cccagagcaa cgccggccct          840

agaccccaca tcggcgacac cctgttcacc ctgtttagag cccccgagct gctggccccc          900

aacggcgacc tgtacaacgt gttcgcctgg gccctggacg tgctggccaa gagactgaga          960

cccatgcacg tgttcatcct ggactacgac cagagccccg ccggctgcag agatgccctg          1020

ctgcagctga ccagcggcat ggtgcagacc cacgtgacca cacccggcag catccccacc          1080

atctgcgacc tggccagaac ctttgccaga gagatgggcg aggccaactg a                1131

<210>    5
<211>    454
<212>    PRT
<213>    Artificial Sequence

<220>
<223>    amino acid sequence of c-Myc

<400>    5

Met Asp Phe Phe Arg Val Val Glu Asn Gln Gln Pro Pro Ala Thr Met
1               5                   10                  15

Pro Leu Asn Val Ser Phe Thr Asn Arg Asn Tyr Asp Leu Asp Tyr Asp
            20                  25                  30

Ser Val Gln Pro Tyr Phe Tyr Cys Asp Glu Glu Glu Asn Phe Tyr Gln
        35                  40                  45

Gln Gln Gln Gln Ser Glu Leu Gln Pro Pro Ala Pro Ser Glu Asp Ile
    50                  55                  60

Trp Lys Lys Phe Glu Leu Leu Pro Thr Pro Pro Leu Ser Pro Ser Arg
65                  70                  75                  80

Arg Ser Gly Leu Cys Ser Pro Ser Tyr Val Ala Val Thr Pro Phe Ser
                85                  90                  95

Leu Arg Gly Asp Asn Asp Gly Gly Gly Gly Ser Phe Ser Thr Ala Asp
            100                 105                 110

Gln Leu Glu Met Val Thr Glu Leu Leu Gly Gly Asp Met Val Asn Gln
        115                 120                 125

Ser Phe Ile Cys Asp Pro Asp Asp Glu Thr Phe Ile Lys Asn Ile Ile
    130                 135                 140

Ile Gln Asp Cys Met Trp Ser Gly Phe Ser Ala Ala Ala Lys Leu Val
145                 150                 155                 160

Ser Glu Lys Leu Ala Ser Tyr Gln Ala Ala Arg Lys Asp Ser Gly Ser
                165                 170                 175

Pro Asn Pro Ala Arg Gly His Ser Val Cys Ser Thr Ser Ser Leu Tyr
            180                 185                 190

Leu Gln Asp Leu Ser Ala Ala Ala Ser Glu Cys Ile Asp Pro Ser Val
        195                 200                 205

Val Phe Pro Tyr Pro Leu Asn Asp Ser Ser Ser Pro Lys Ser Cys Ala
    210                 215                 220

Ser Gln Asp Ser Ser Ala Phe Ser Pro Ser Ser Asp Ser Leu Leu Ser
225                 230                 235                 240

Ser Thr Glu Ser Ser Pro Gln Gly Ser Pro Glu Pro Leu Val Leu His
                245                 250                 255

Glu Glu Thr Pro Pro Thr Thr Ser Ser Asp Ser Glu Glu Glu Gln Glu
            260                 265                 270

Asp Glu Glu Glu Ile Asp Val Val Ser Val Glu Lys Arg Gln Ala Pro
        275                 280                 285

Gly Lys Arg Ser Glu Ser Gly Ser Pro Ser Ala Gly Gly His Ser Lys
    290                 295                 300

Pro Pro His Ser Pro Leu Val Leu Lys Arg Cys His Val Ser Thr His
305                 310                 315                 320

Gln His Asn Tyr Ala Ala Pro Pro Ser Thr Arg Lys Asp Tyr Pro Ala
                325                 330                 335

Ala Lys Arg Val Lys Leu Asp Ser Val Arg Val Leu Arg Gln Ile Ser
            340                 345                 350

Asn Asn Arg Lys Cys Thr Ser Pro Arg Ser Ser Asp Thr Glu Glu Asn
        355                 360                 365

Val Lys Arg Arg Thr His Asn Val Leu Glu Arg Gln Arg Arg Asn Glu
    370                 375                 380

Leu Lys Arg Ser Phe Phe Ala Leu Arg Asp Gln Ile Pro Glu Leu Glu
385                 390                 395                 400

Asn Asn Glu Lys Ala Pro Lys Val Val Ile Leu Lys Lys Ala Thr Ala
                405                 410                 415

Tyr Ile Leu Ser Val Gln Ala Glu Glu Gln Lys Leu Ile Ser Glu Glu
            420                 425                 430

Asp Leu Leu Arg Lys Arg Arg Glu Gln Leu Lys His Lys Leu Glu Gln
        435                 440                 445

Leu Arg Asn Ser Cys Ala
    450


<210>    6
<211>    1365
<212>    DNA
<213>    Artificial Sequence

<220>
<223>    nucleotide sequence of c-Myc


<400>    6
atggatttct ttcgcgtcgt cgagaaccag cagccacccg ccactatgcc tctgaacgtg      60

tcttttacta acaggaacta tgatctggat tacgacagcg tgcagcccta cttctattgc     120

gatgaggaag agaactttta tcagcagcag cagcagagcg agctgcagcc acctgcacct     180

tccgaagaca tttggaagaa attcgagctg ctgcctacac cacccctgtc tccaagtcgg     240

agaagcggcc tgtgttcacc cagctacgtg gccgtcactc ctttcagcct gaggggggac     300

aatgatggcg ggggaggctc cttttctaca gccgatcagc tggaaatggt gactgagctg     360

ctggggggag acatggtcaa ccagagcttc atttgcgatc ctgacgatga aacttttatc     420

aagaacatca tcatccagga ctgtatgtgg tcaggcttta gcgccgctgc aaagctggtg     480

tctgagaaac tggcaagtta tcaggccgct cggaaagata gtgggtcacc taacccagct     540

agaggacact ccgtgtgctc tacaagctcc ctgtacctgc aggacctgag cgcagccgct     600

tccgagtgta ttgatccctc cgtggtcttc ccctatcctc tgaatgactc tagttcaccc     660

aagagttgcg catcacagga cagctccgcc ttttcacctt ctagtgatag cctgctgtca     720

agcactgaat cctctccaca gggcagccca gagccactgg tgctgcatga agagacccct     780

ccaaccacaa gttcagattc cgaagaggaa caggaggacg aggaagagat cgatgtggtc     840

tctgtggaaa agcgccaggc tccaggaaaa cgaagcgagt ccggctctcc aagtgcagga 900

ggacactcca agccacctca ttctcccctg gtgctgaaaa ggtgccacgt ctccacccac 960

cagcataact acgcagcccc accctctaca cgaaaggact atccagctgc aaagcgcgtg 1020

aaactggata gcgtgagagt cctgaggcag atcagtaaca atcggaagtg tacttcaccc 1080

agaagctccg acaccgaaga gaacgtgaaa aggcgcaccc ataatgtcct ggaacgccag 1140

cgacggaatg agctgaagag gtccttcttt gccctgcgcg atcagattcc tgaactggag 1200

aacaatgaga aggctccaaa agtggtcatt ctgaagaaag ccacagctta catcctgtct 1260

gtgcaggccg aagagcagaa actgatcagt gaagaggacc tgctgagaaa acgcagggaa 1320

cagctgaaac ataaactgga acagctgaga aactcttgtg cttaa 1365

<210> 7
<211> 1132
<212> PRT
<213> Artificial Sequence

<220>
<223> amino acid sequence of hTERT

<400> 7


<210>   8
<211>   3399
<212>   DNA
<213>   Artificial Sequence

<220>
<223>   nucleotide sequence of hTERT

<400> 8
atgcccagag ctcccagatg cagagccgtg agaagcctgc tgagaagcca ctacagagag 60

gtgctgcccc tggccacctt cgtgagaaga ctgggacccc agggctggag actggtgcag 120

agaggcgacc ccgcagcctt tagagccctg gtggcccagt gcctggtgtg cgtgccctgg 180

gacgccagac ctcctcccgc tgcccccagc ttcagacagg tgagctgcct gaaggagctg 240

gtggccagag tgctccagag actgtgcgag agaggcgcca agaacgtgct ggcctttggc 300

ttcgccctgc tggatggagc cagaggcgga cctcccgagg ccttcaccac aagcgtgaga 360

agctacctgc ccaacaccgt gaccgatgcc ctgagaggct ccggcgcctg gggcctgctc 420

ctgagaagag tgggcgacga cgtgctggtg cacctgctgg ccagatgcgc cctgttcgtg 480

ctggtggctc ccagctgcgc ctaccaggtg tgcggacccc ctctgtacca gctgggagcc 540

gccacccagg caagaccccc tccccacgcc tctggaccca gaagaagact gggctgcgag 600

agagcctgga accacagcgt gagagaggct ggcgtgcccc tgggcctgcc cgcccctggc 660

gccagaagaa gaggcggcag cgccagcaga agcctgcccc tgcccaagag acccagacgc 720

ggcgccgctc ccgagcctga gagaacaccc gtgggccagg gcagctgggc ccaccccggc 780

agaaccagag gacccagcga cagaggcttc tgcgtggtga gccctgccag acccgccgag 840

gaggccacca gcctggaggg cgccctgagc ggcaccagac acagccaccc cagcgtgggc 900

agacagcacc acgccggccc tcctagcacc agcagacccc ccagaccttg ggacaccccc 960

tgcccccctg tgtacgccga gaccaagcac ttcctgtaca gcagcggcga caaggagcag 1020

ctgagaccca gcttcctgct gagctccctg agacccagcc tgaccggcgc cagaagactg 1080

gtggagacca tcttcctggg cagcagaccc tggatgcccg gcacccccag aagactgccc 1140

agactgcccc agagatactg gcagatgaga cccctgttcc tggagctgct gggcaaccac 1200

gcccagtgcc cctacggcgt gctgctgaag acccactgcc ccctgagagc tgccgtgacc 1260

cccgcagctg gcgtgtgcgc cagagagaag ccccagggca gcgtggccgc tcccgaggag 1320

gaggacaccg atcccagaag actggtgcag ctgctgagac agcacagcag cccctggcag 1380

gtgtacggct tcgtgagagc ctgcctgaga agactggtgc ctcccggcct gtggggcagc 1440

agacacaacg agagaagatt cctgagaaac accaagaagt tcatcagcct gggcaagcac 1500

gccaagctga gcctccagga gctgacatgg aagatgagcg tgagagactg cgcctggctg 1560

aggagaagcc ctggcgtggg ctgcgtgccc gccgccgagc acagactgag agaggagatc 1620

ctggccaagt ttctgcactg gctgatgagc gtgtacgtgg tggagctgct gagaagcttc 1680

ttctacgtga ccgagaccac attccagaag aacagactgt tcttttacag gaagagcgtg 1740

tggagcaagc tccagagcat cggcatcaga cagcacctga agagagtgca gctgagagag 1800

ctgagcgagg ccgaggtgag acagcacaga gaggccagac ccgccctgct gaccagcaga 1860

ctgagattca tccccaagcc cgatggcctg agacccatcg tgaacatgga ctacgtggtg 1920

ggagccagaa cctttagaag agagaagaga gccgagagac tgaccagcag agtgaaggcc 1980

ctgttcagcg tgctgaacta cgagagagcc agaagacccg gcctgctggg cgccagcgtg 2040

ctgggcctgg acgacatcca cagagcctgg agaaccttcg tgctgagagt gagagcccag 2100

gaccctcctc ccgagctgta cttcgtgaag gtggacgtga ccggcgccta cgacaccatc 2160

ccccaggaca gactgaccga ggtgatcgcc agcatcatca agccccagaa cacctactgc 2220

gtgagaagat acgccgtggt gcagaaggcc gcccacggcc acgtgagaaa ggccttcaag 2280

agccacgtga gcaccctgac cgacctccag ccctacatga gacagttcgt ggcccacctc 2340

caggagacca gccccctgag agatgccgtg gtgatcgagc agagctcttc cctgaacgag 2400

gcctccagcg gcctgttcga cgtgttcctg agattcatgt gccaccacgc cgtgagaatc 2460

agaggcaaga gctacgtgca gtgccagggc atcccccagg gcagcatcct gagcaccctg 2520

ctgtgcagcc tgtgctacgg cgacatggag aacaagctgt tcgctggcat cagaagagac 2580

ggcctgctgc tgagactggt ggacgacttc ctgctggtga ccccccacct gacccacgcc 2640

aagaccttcc tgagaaccct ggtgagaggc gtgcccgagt acggctgcgt ggtgaacctg 2700

agaaagaccg tggtgaactt tcccgtggag gacgaggccc tgggcggcac cgccttcgtg 2760

cagatgcccg cccacggcct gtttccctgg tgcggcctgc tcctcgacac cagaaccctg 2820

gaggtgcaga gcgactacag cagctacgca agaaccagca tcagagccag cctgaccttc 2880

aacagaggct tcaaggccgg cagaaacatg agaagaaagc tgttcggcgt gctgagactg 2940

aagtgccaca gcctgttcct ggacctccag gtgaacagcc tccagaccgt gtgcaccaac 3000

atctacaaga tcctgctgct ccaggcctac agattccacg cctgcgtgct ccagctgccc 3060

ttccaccagc aggtgtggaa gaatcccacc ttcttcctga gagtgatcag cgacaccgcc 3120

agcctgtgct acagcatcct gaaggccaag aatgccggca tgagcctggg cgccaagggc 3180

gccgctggac ccctgcccag cgaggccgtg cagtggctgt gccaccaggc cttcctgctg 3240

aagctgacca gacacagagt gacctacgtg cccctgctgg gcagcctgag aaccgcccag 3300

acccagctga gcagaaagct gcctggcaca accctgaccg ccctggaggc agccgcaaac 3360

cccgccctgc ccagcgactt caagaccatc ctggactag 3399

<210> 9
<211> 248
<212> PRT
<213> Artificial Sequence

<220>
<223> amino acid sequence of tTA

<400> 9

Met Ser Arg Leu Asp Lys Ser Lys Val Ile Asn Ser Ala Leu Glu Leu
1               5                   10                  15

Leu Asn Glu Val Gly Ile Glu Gly Leu Thr Thr Arg Lys Leu Ala Gln
            20                  25                  30

Lys Leu Gly Val Glu Gln Pro Thr Leu Tyr Trp His Val Lys Asn Lys
        35                  40                  45

Arg Ala Leu Leu Asp Ala Leu Ala Ile Glu Met Leu Asp Arg His His
    50                  55                  60

Thr His Phe Cys Pro Leu Glu Gly Glu Ser Trp Gln Asp Phe Leu Arg
65                  70                  75                  80

Asn Asn Ala Lys Ser Phe Arg Cys Ala Leu Leu Ser His Arg Asp Gly
                85                  90                  95

Ala Lys Val His Leu Gly Thr Arg Pro Thr Glu Lys Gln Tyr Glu Thr
            100                 105                 110

Leu Glu Asn Gln Leu Ala Phe Leu Cys Gln Gln Gly Phe Ser Leu Glu
        115                 120                 125

Asn Ala Leu Tyr Ala Leu Ser Ala Val Gly His Phe Thr Leu Gly Cys
    130                 135                 140

Val Leu Glu Asp Gln Glu His Gln Val Ala Lys Glu Glu Arg Glu Thr
145                 150                 155                 160

Pro Thr Thr Asp Ser Met Pro Pro Leu Leu Arg Gln Ala Ile Glu Leu
                165                 170                 175

Phe Asp His Gln Gly Ala Glu Pro Ala Phe Leu Phe Gly Leu Glu Leu
            180                 185                 190

Ile Ile Cys Gly Leu Glu Lys Gln Leu Lys Cys Glu Ser Gly Gly Pro
        195                 200                 205

Ala Asp Ala Leu Asp Asp Phe Asp Leu Asp Met Leu Pro Ala Asp Ala
    210                 215                 220

Leu Asp Asp Phe Asp Leu Asp Met Leu Pro Ala Asp Ala Leu Asp Asp
225                 230                 235                 240

Phe Asp Leu Asp Met Leu Pro Gly
                245


<210>   10
<211>   747
<212>   DNA
<213>   Artificial Sequence

<220>
<223>   nucleotide sequence of tTA


<400>   10
atgtcaaggc tggataaaag caaagtgatt aactccgctc tggaactgct gaacgaagtc      60

ggcattgagg ggctgaccac acgcaagctg gcacagaagc tgggagtgga gcagcccacc      120

ctgtactggc acgtgaagaa caagcgcgcc ctgctggacg ccctggccat cgagatgctg 180

gatcggcacc acacacactt ctgccctctg gagggcgaga gctggcagga cttcctgcgg 240

aacaatgcca agagctttag atgtgccctg ctgtcccaca gggatggagc aaaggtgcac 300

ctgggcacca gaccaacaga gaagcagtac gagaccctgg agaaccagct ggccttcctg 360

tgccagcagg gcttttctct ggagaatgcc ctgtatgccc tgagcgccgt gggacacttc 420

accctgggat gcgtgctgga ggaccaggag caccaggtgg ccaaggagga gagagagaca 480

cctaccacag actccatgcc ccctctgctg aggcaggcca tcgagctgtt tgatcaccag 540

ggcgccgagc cagccttcct gtttggcctg gagctgatca tctgcggcct ggagaagcag 600

ctgaagtgtg agtctggagg accagcagat gccctggacg atttcgacct ggatatgctg 660

cccgccgacg ccctggacga ttttgatctg gacatgctgc ctgctgatgc cctggatgat 720

tttgacctgg atatgctgcc tggataa 747

<210> 11
<211> 199
<212> PRT
<213> Artificial Sequence

<220>
<223> amino acid sequence of PuroR

<400> 11
Met Thr Glu Tyr Lys Pro Thr Val Arg Leu Ala Thr Arg Asp Asp Val
1               5                   10                  15

Pro Arg Ala Val Arg Thr Leu Ala Ala Ala Phe Ala Asp Tyr Pro Ala
            20                  25                  30

Thr Arg His Thr Val Asp Pro Asp Arg His Ile Glu Arg Val Thr Glu
        35                  40                  45

Leu Gln Glu Leu Phe Leu Thr Arg Val Gly Leu Asp Ile Gly Lys Val
    50                  55                  60

Trp Val Ala Asp Asp Gly Ala Ala Val Ala Val Trp Thr Thr Pro Glu
65                  70                  75                  80

Ser Val Glu Ala Gly Ala Val Phe Ala Glu Ile Gly Pro Arg Met Ala
                85                  90                  95

Glu Leu Ser Gly Ser Arg Leu Ala Ala Gln Gln Gln Met Glu Gly Leu
            100                 105                 110

Leu Ala Pro His Arg Pro Lys Glu Pro Ala Trp Phe Leu Ala Thr Val
        115                 120                 125

Gly Val Ser Pro Asp His Gln Gly Lys Gly Leu Gly Ser Ala Val Val
    130                 135                 140

Leu Pro Gly Val Glu Ala Ala Glu Arg Ala Gly Val Pro Ala Phe Leu
145                 150                 155                 160

Glu Thr Ser Ala Pro Arg Asn Leu Pro Phe Tyr Glu Arg Leu Gly Phe
                165                 170                 175

Thr Val Thr Ala Asp Val Glu Val Pro Glu Gly Pro Arg Thr Trp Cys
            180                 185                 190

Met Thr Arg Lys Pro Gly Ala
        195

<210>    12
<211>    600
<212>    DNA
<213>    Artificial Sequence

<220>
<223>    nucleotide sequence of PuroR

<400>    12
atgaccgagt acaagcccac ggtgcgcctc gccacccgcg acgacgtccc cagggccgta      60

cgcaccctcg ccgccgcgtt cgccgactac cccgccacgc gccacaccgt cgatccggac     120

cgccacatcg agcgggtcac cgagctgcaa gaactcttcc tcacgcgcgt cgggctcgac     180

atcggcaagg tgtgggtcgc ggacgacggc gccgcggtgg cggtctggac cacgccggag     240

agcgtcgaag cgggggcggt gttcgccgag atcggcccgc gcatggccga gttgagcggt     300

tcccggctgg ccgcgcagca acagatggaa ggcctcctgg cgccgcaccg gcccaaggag     360

cccgcgtggt tcctggccac cgtcggcgtc tcgcccgacc accagggcaa gggtctgggc     420

agcgccgtcg tgctccccgg agtggaggcg gccgagcgcg ccggggtgcc cgccttcctg     480

gagacctccg cgccccgcaa cctccccttc tacgagcggc tcggcttcac cgtcaccgcc     540

gacgtcgagg tgcccgaagg accgcgcacc tggtgcatga cccgcaagcc cggtgcctga     600

                                                                      600

<210>    13
<211>    124
<212>    PRT
<213>    Artificial Sequence

<220>
<223>    amino acid sequence of ZeoR

<400>    13
Met Ala Lys Leu Thr Ser Ala Val Pro Val Leu Thr Ala Arg Asp Val
  1               5                  10                  15

Ala Gly Ala Val Glu Phe Trp Thr Asp Arg Leu Gly Phe Ser Arg Asp
             20                  25                  30

Phe Val Glu Asp Asp Phe Ala Gly Val Val Arg Asp Asp Val Thr Leu
         35                  40                  45

Phe Ile Ser Ala Val Gln Asp Gln Val Val Pro Asp Asn Thr Leu Ala

50                  55                  60

Trp Val Trp Val Arg Gly Leu Asp Glu Leu Tyr Ala Glu Trp Ser Glu
65                  70                  75                  80

Val Val Ser Thr Asn Phe Arg Asp Ala Ser Gly Pro Ala Met Thr Glu
                85                  90                  95

Ile Gly Glu Gln Pro Trp Gly Arg Glu Phe Ala Leu Arg Asp Pro Ala
            100                 105                 110

Gly Asn Cys Val His Phe Val Ala Glu Glu Gln Asp
        115                 120


<210>    14
<211>    375
<212>    DNA
<213>    Artificial Sequence

<220>
<223>    nucleotide sequence of ZeoR


<400>    14
atggccaagt tgaccagtgc cgttccggtg ctcaccgcgc gcgatgtggc cggagcggtc      60

gagttctgga ccgaccggct cgggttcagc cgggacttcg tggaggacga cttcgccggt     120

gtggtccggg acgacgtgac cctgttcatc agcgcggtcc aggaccaggt ggtgccggac     180

aacaccctgg cctgggtgtg ggtgcgcggc ctggacgagc tgtacgccga gtggtcggag     240

gtcgtgtcca cgaacttccg ggacgcctcc gggccggcca tgaccgagat cggcgagcag     300

ccgtgggggc gggagttcgc cctgcgcgac ccggccggca actgcgtgca cttcgtggcc     360

gaggagcagg actaa                                                      375


<210>    15
<211>    264
<212>    PRT
<213>    Artificial Sequence

<220>
<223>    amino acid sequence of NeoR


<400>    15

<210>   16
<211>   795
<212>   DNA
<213>   Artificial Sequence

<220>
<223>   nucleotide sequence of NeoR

<400>   16
atgattgaac aagatggatt gcacgcaggt tctccggccg cttgggtgga gaggctattc      60

ggctatgact gggcacaaca gacaatcggc tgctctgatg ccgccgtgtt ccggctgtca     120

gcgcaggggc gcccggttct ttttgtcaag accgacctgt ccggtgccct gaatgaactg     180

caggacgagg cagcgcggct atcgtggctg gccacgacgg gcgttccttg cgcagctgtg     240

ctcgacgttg tcactgaagc gggaagggac tggctgctat tgggcgaagt gccggggcag     300

gatctcctgt catctcacct tgctcctgcc gagaaagtat ccatcatggc tgatgcaatg     360

cggcggctgc atacgcttga tccggctacc tgcccattcg accaccaagc gaaacatcgc     420

atcgagcgag cacgtactcg gatggaagcc ggtcttgtcg atcaggatga tctggacgaa 480

gagcatcagg ggctcgcgcc agccgaactg ttcgccaggc tcaaggcgcg catgcccgac 540

ggcgaggatc tcgtcgtgac ccatggcgat gcctgcttgc cgaatatcat ggtggaaaat 600

ggccgctttt ctggattcat cgactgtggc cggctgggtg tggcggaccg ctatcaggac 660

atagcgttgg ctacccgtga tattgctgaa gagcttggcg gcgaatgggc tgaccgcttc 720

ctcgtgcttt acggtatcgc cgctcccgat tcgcagcgca tcgccttcta tcgccttctt 780

gacgagttct tctaa 795

## Description

### [Technical Field]

The present application claims priority to and the benefit of Korean Patent Application No. 10-2018-0008569 filed in the Korean Intellectual Property Office on January 24, 2018, the entire contents of which are incorporated herein by reference.

The present invention relates to a recombinant lentiviral vector comprising a gene encoding a brain-derived neurotrophic factor (BDNF) protein, and a cell transfected with a lentivirus prepared using the vector.

### [Background Art]

A brain-derived neurotrophic factor (BDNF) is expressed in our body's brain, retina, motor neurons, kidneys, saliva, prostate, and the like, and in the hippocampus, the cerebral cortex, the hypothalamus, the basal forebrain, and the like. As BDNF plays an important part in long-term memory, BDNF mRNA is expressed at a high level in the hippocampal region in a learning animal. It is reported that an increase in expression in such a hippocampal region is also associated with learning levels.

Further, BDNF is a material showing the best activity in neurotrophins, which promote the generation of nerve cells. In fact, a mouse in which BDNF was genetically completely eliminated showed severe postnatal mortality, and the loss of nerve cells is observed in the cerebellum, the olfactory bulb, the visual cortex, the cochlear, and the like. In contrast, when the BDNF gene is partially removed, neurotrophin expression is reduced, long-term potentiation (LTP) of the hippocampal region and changes in sensory and pain systems are observed, and behavioral problems as well as psychological problems are observed.

Meanwhile, BDNF is utilized as a target material in the development of drugs for treating a neurodegenerative disorder. Experimental evidence suggesting that BDNF is involved in neurodegenerative diseases such as Alzheimer's disease (AD), Parkinson's disease (PD), Huntington's disease (HD), and amyotrophic lateral sclerosis (ALS) has been reported since the early 1990s. Various studies have reported that BDNF genetic abnormalities are associated with diseases. In fact, in patients suffering from such a neurodegenerative disease, the amount of BDNF is lower than that in normal people. In particular, in regard to Huntington's disease, it was reported through various cell and animal experiments that a BDNF transport problem occurs as the disease progresses. Attempts have been made to treat the disease by a method of supplying BDNF from the outside, but to date, there is a problem that it is difficult to effectively deliver BDNF to the brain.

Meanwhile, a therapeutic method using cells has been developed worldwide, and many studies have been conducted on a cell therapeutic agent using adult stem cells. Mesenchymal stem cells (MSCs), which are adult stem cells, are multipotent cells that can differentiate into bone, cartilage, muscle, fat, fibroblasts, and the like. MSCs can be relatively easily obtained from various adult tissues such as bone marrow, cord blood, and fat. MSCs have the specificity of migrating to the site of inflammation or injury, and thus have a big advantage as a vehicle for delivering a therapeutic drug. Further, MSCs can suppress or activate the functions of immune cells such as T cells, B cells, dendritic cells and natural killer cells to regulate the immune function of the human body. In addition, MSCs have an advantage in that they can be cultured relatively easily *in vitro.* Due to such characteristics, studies have been actively conducted to use the MSC as a cell therapeutic agent.

However, despite such advantages of MSCs, there are the following problems in producing MSCs at a clinically usable level as a cell therapeutic agent. First, since there are limits in the proliferation of MSCs, it is difficult to produce MSCs in large amounts. Second, since the obtained MSCs are mixed with various types of cells, it is difficult to maintain the same effect during production. Third, the therapeutic effect is not enough when only MSCs are used. Finally, MSCs injected into the human body are also likely to become cancer cells in the body.

Meanwhile, Korean Patent No. 1585032 discloses a cell therapeutic agent containing mesenchymal stem cells cultured in a hydrogel. The document provides a composition which can be ready to administer by shortening the pretreatment process in the step of isolating mesenchymal stem cells for use as a cell therapeutic agent, but the problems of the mesenchymal stem cells as described above and a solution for solving the problems are not mentioned at all. Therefore, there is a need for a study on safe and effective mesenchymal stem cells that can be usefully used as a cell therapeutic agent.

In particular, a study has been conducted to transform mesenchymal stem cells using an adenovirus so as to express a brain-derived neurotrophic factor (BDNF) (Kari Pollock et al., Molecular Therapy, vol.24 no.5: 965-977, 2016). However, the mesenchymal stem cells used in the study are general mesenchymal stem cells obtained from tissues, and since the proliferation of MSCs is limited, there is a problem in that it is difficult to mass-produce mesenchymal stem cells. In addition, the expression level of the BDNF gene is various, so that it is difficult to expect the same effect during production.

### [Disclosure]

### [Technical Problem]

An object of the present invention is to provide a transfected host cell that can be subcultured (proliferated) for 160 days or longer to develop a stem cell therapeutic agent capable of being mass-produced and maintaining the same effect.

Another object of present invention is to provide a transfected host cell that can be stably express the BDNF gene for 160 days or longer and show effects of protecting and regenerating nerve cells.

### [Technical Solution]

To achieve the object, the present invention provides a recombinant lentiviral vector including a gene encoding a BDNF protein.

Further, the present invention provides a recombinant lentivirus including a gene encoding the BDNF protein.

Further, the present invention provides a host cell transfected with the recombinant lentivirus.

Further, the present invention provides a pharmaceutical composition for preventing or treating a neurological disease, including the recombinant lentivirus as an active ingredient.

Further, the present invention provides a pharmaceutical composition for preventing or treating a neurological disease, including the transfected host cell as an active ingredient.

Further, the present invention provides a method for preventing or treating a neurological disease, the method including: administering the recombinant lentivirus or the host cell transfected with the recombinant lentivirus to a subject.

In addition, the present invention provides a use of the recombinant lentivirus or the host cell transfected with the recombinant lentivirus for the prevention or treatment of a neurological disease.

### [Advantageous Effects]

A mesenchymal stem cell expressing the BDNF protein of the present invention and a pharmaceutical composition for preventing or treating a neurological disease, including the same as an active ingredient, express BDNF as a stem cell therapeutic agent capable of being mass-produced and maintaining the same effect to induce the protection and regeneration of nerve cells, thereby promoting the formation of brain tissue and improving the function of the nervous system. Further, the mesenchymal stem cell of the present invention is immortalized mesenchymal stem cells, and is highly unlikely to undergo abnormal differentiation and proliferation while having a high cell proliferation rate, and thus is highly safe. Therefore, the pharmaceutical composition of the present invention can be useful as a pharmaceutical composition for preventing or treating a neurological disease.

### [Description of Drawings]

FIG. 1 is a graph comparing the cell proliferation rates of immortalized MSC (imMSC) and normal MSC (MSC):
   X-axis: Culture period; and
   Y-axis: Cumulative population doubling level (PDL).
FIG. 2 is a graph illustrating BDNF protein expression levels of clones #1 to #50 infected Immortalized MSCs transduced with a lentivirus including BDNF gene.
FIG. 3 is a graph illustrating expression levels of BDNF protein according to the presence or absence of doxycycline in cell clones having an excellent expression level of BDNF protein
FIG. 4 is a view illustrating the results of chromosome analysis of cell clones #14, #22, #23 and #41, which are excellent in BDNF protein production.
FIG. 5 is a graph illustrating the cumulative population doubling level for 140 days in clones #14, #22, #23 and #41 selected from strains producing the BDNF protein.
FIG. 6 illustrates the comparison of the expression of surface antigen proteins such as CD90, CD44, CD105, and CD73, which are the inherent properties of MSCs after genetic modification of the BM-01A cell line and of the normal MSCs.
FIG. 7 is a graph illustrating the cell proliferation rate of immortalized MSCs transduced with a lentivirus including the BDNF gene:
   X-axis: Culture period; and
   Y-axis: Cumulative population doubling level (PDL).
FIG. 8 is a view illustrating that cell characteristics are maintained morphologically during a subculture of the BM-01A cell line for 140 days.
FIG. 9 is a graph illustrating the BDNF protein expression level in the BM-01A cell line depending on the presence or absence of doxycycline treatment.
FIG. 10 is a view illustrating that the BDNF protein expression level was maintained constantly for each subculture during the subculture of the BM-01A cell line.
FIG. 11 is a view illustrating that confirm of BDNF gene has been introduced into the BM-01A cell line.
FIG. 12 is a view illustrating the change in volume of a Huntington's disease-induced rat lateral ventricle zone (LVZ) according to the administration of quinolinic acid (QA).
FIG. 13 is a view illustrating whether or not the BM-01A cell line is present in the brain one week after the administration of the BM-01A cell line into the brain of a Huntington's disease-induced rat.
FIG. 14 is a graph illustrating the behavioral improvement effect in Huntington's disease-induced rats according to the administration of the BM-01A cell line by a Rota-rod test.
FIG. 15 is a graph illustrating the behavioral improvement effect of Huntington's disease-induced rats according to the administration of the BM-01A cell line by a stepping test.
FIG. 16 is a graph illustrating the behavioral improvement effect in Huntington's disease-induced rats according to the administration of the BM-01A cell line by a staircase test.
FIG. 17 is a set of photographs illustrating the immunostaining of Iba-1 and ED1 in the brain tissue of Huntington's disease-induced rats after administration of a cryo-buffer or the BM-01A cell line.
FIG. 18 is a set of photographs illustrating the immunostaining of ED1 and an inflammation marker iNOS in the brain tissue of Huntington's disease-induced rats after administration of a cryo-buffer or the BM-01A cell line into cells.
FIG. 19 is a set of photographs illustrating the immunostaining of glial cells in the brain tissue of Huntington's disease-induced rats after administration of a cryo-buffer or the BM-01A cell line.
FIG. 20 is a photograph illustrating the LVZ volume of Huntington's disease-induced rats after administration of a cryo-buffer or the BM-01A cell line.
FIG. 21 is a graph illustrating the LVZ volume of Huntington's disease-induced rats after administration of a cryo-buffer or the BM-01A cell line.
FIG. 22 is a photograph illustrating deletion parts in the striatum of Huntington's disease-induced rats after administration of a cryo-buffer or the BM-01A cell line.
FIG. 23 is a graph illustrating deletion parts in the striatum of Huntington's disease-induced rats after administration of a cryo-buffer or the BM-01A cell line.
FIG. 24 is a graph illustrating the density in the striatum of Huntington's disease-induced rats after administration of a cryo-buffer or the BM-01A cell line.
FIG. 25 is a photograph illustrating the immunostaining of DCX in the brain tissue of Huntington's disease-induced rats after administration of a cryo-buffer or the BM-01A cell line.
FIG. 26 is a graph illustrating the behavioral improvement effect in stroke-induced rats according to the administration of the BM-01A cell line by a Rota-rod test.
FIG. 27 is a graph illustrating the behavioral improvement effect of stroke-induced rats according to the administration of the BM-01A cell line by a stepping test.
FIG. 28 is a graph illustrating the behavioral improvement effect of stroke-induced rats according to the administration of the BM-01A cell line by a modified neurological severity score test.
FIG. 29 is a set of photographs illustrating the staining of the glial scars of stroke-induced rats after administration of a cryo-buffer (vehicle) or the BM-01A cell line.
FIG. 30 is a graph illustrating the decreases in scar area in the cortical and striatal regions of stroke-induced rats after administration of a cryo-buffer (vehicle) or the BM-01A cell line

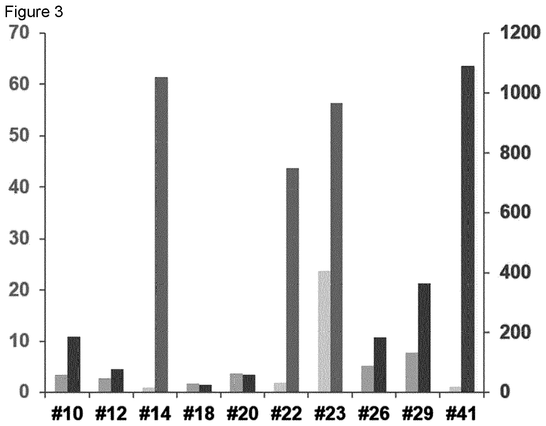

FIG. 3 1 is a graph illustrating the decrease in scar thickness in the cortical and striatal regions of stroke-induced rats after administration of a cryo-buffer (vehicle) or the BM-01A cell line.

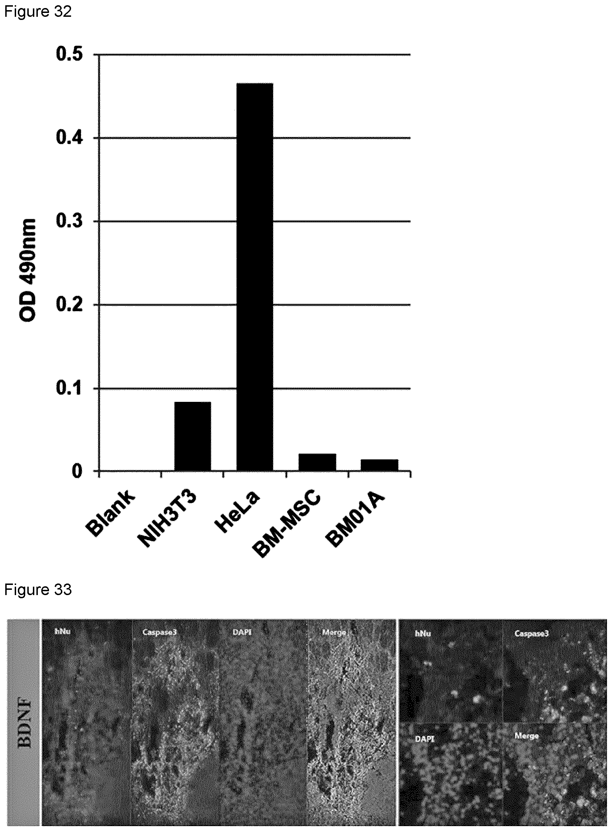

FIG. 32 is a graph illustrating that there is no oncogenicity even after genetic manipulation of the BM-01A cell line.
FIG. 33 is a view illustrating whether the BM-01A cell line in the brain tissues of Huntington's disease-induced rats at 10 weeks after administration of the BM-01A cell line survives.

### [Modes of the Invention]

Hereinafter, the present invention will be described in detail.

The present invention provides a recombinant lentiviral vector including a gene encoding a BDNF protein.

As used herein, the term "brain-derived neurotrophic factor (BDNF)" protein refers to a neurotrophin distributed most abundantly in the brain. The BDNF protein is known to promote the growth of a neuron and regulate the synthesis, metabolism, and release of a neurotransmitter and the activity of the neuron. Further, it is known that the BDNF protein is reduced in the frontal cortex or hippocampus of a patient with depression and it can be treated by increasing the concentration of the BDNF protein.

The BDNF protein according to the present invention may be a human-derived protein. The BDNF protein of the present invention may be a polypeptide having the amino acid sequence of SEQ ID NO: 1. The BDNF protein may have about 80%, 90%, 95%, or 99% or higher homology with the amino acid sequence of SEQ ID NO: 1. Meanwhile, the gene encoding the BDNF protein may be a polynucleotide having nucleotide sequence of SEQ ID NO: 2. In addition, the nucleotide sequence encoding the BDNF protein may have about 80%, 90%, 95% or 99% or higher homology with the nucleotide sequence of SEQ ID NO: 2.

As used herein, the term "lentiviral vector" is a kind of retrovirus. The vector is also referred to interchangeably as "lentiviral transfer vector". The lentiviral vector can be inserted into the genomic DNA of a target cell to stably express the gene, and can transfer the gene to the mitotic and non-mitotic cells. Since the vector does not induce an immune response of a human body, its expression is continuous. In addition, there is an advantage that gene of a large size can be delivered as compared to an adenovirus vector which is a conventional virus vector. The recombinant lentiviral vector of the present invention can regulate gene expression by a promoter. The promoter may be a cytomegalovirus (CMV), respiratory syncytial virus (RSV), human elongation factor-1 alpha (EF-1α) or tetracycline response elements (TRE) promoter. According to one embodiment, the recombinant lentiviral vector can regulate the expression of the BDNF protein by single promoter. The promoter can be operably linked to a gene encoding a protein to be expressed.

According to one embodiment, the BDNF protein may be linked to a TRE promoter. The TRE promoter can activate the transcription of a gene linked to a promoter by a tetracycline transactivator (tTA) protein. Specifically, the tTA protein binds to the TRE promoter and activates transcription when tetracycline or doxycycline is not present, whereas it cannot bind to the TRE promoter and activate the transcription when tetracycline or doxycycline is present. Thus, the expression of the BDNF protein can be regulated by the addition or depletion of tetracycline or doxycycline.

The term "operably linked" refers that a particular polynucleotide is linked to another polynucleotide so that it can conduct its function. The expression that a gene encoding a particular protein is operably linked to a promoter implies that the gene can be transcribed into mRNA by the action of the promoter and translated into a protein.

The present invention provides a recombinant lentivirus including a gene encoding the BDNF protein.

The recombinant lentivirus may be obtained by the steps of transforming a host cell with the lentiviral vector of the present invention, a packaging plasmid, and an envelope plasmid; and isolating the lentivirus from the transformed host cell.

The terms "packaging plasmid" and "envelope plasmid" are plasmids including a gene encoding a protein and may provide a helper constructs (e.g., a plasmid or separated nucleic acid) for producing lentiviruses except the lentiviral vectors of the present invention for effective transfection. Such constructs contain useful elements for preparing and packaging lentiviral vectors in host cells. The above elements include a structural protein such as a GAG precursor; a processing protein such as a pol precursor; a protease, an envelope protein; and expression and regulatory signals necessary to synthesize protein and produce lentiviral particles in the host cell, etc.

For production of the recombinant lentivirus, Lenti-X Lentiviral Expression System provided by Clonetech Laboratories Inc., a packaging plasmid(e.g., pRSV-Rev, psPAX, pCl-VSVG, pNHP, etc.) or an envelope plasmid(e.g., pMD2.G, pLTR-G, pHEF-VSVSVG, etc.) provided by Addgene can be used.

Further, the present invention provides a host cell transfected with the above recombinant lentivirus.

The term "transduction" refers to the delivery of a genes loaded in a recombinant lentiviral vector via viral infection.

A host cell according to the present invention may be a human embryonic stem cell (hES), a bone marrow stem cell (BMSC), a mesenchymal stem cell (MSC), a human neural stem cell (hNSC), a limbal stem cell or an oral mucosal epithelial cell. According to one embodiment of the present invention, the host cell may be a mesenchymal stem cell.

The term "mesenchymal stem cell (MSC)" refers to a multipotent stromal cell capable of differentiating into various cells including osteocytes, chondrocytes and adipocytes. Mesenchymal stem cell can differentiate into cells of specific organs such as a bone, a cartilage, a fat, a tendon, a nervous tissue, fibroblasts and myocytes. These cells can be isolated or purified from adipose tissues, bone marrows, peripheral blood, umbilical cord blood, periosteum, dermis, mesodermal-derived tissues and the like.

The transfected host cell according to the present invention may be immortalized by hTERT and/or c-Myc gene.

Further, the transfected host cell according to the present invention may further include a thymidine kinase (TK) gene.

The term "thymidine kinase" refers to an enzyme that catalyzes thymidylic acid production reaction by transferring phosphoric acid from the γ-position of ATP to thymidine, thymidine is transformed into a triphosphate form. The modified thymidine cannot be used for DNA replication, and is known to induce death of cells containing it. TK proteins can be used as long as they have a known sequence. According to an exemplary embodiment, the TK protein may be a polypeptide having an amino acid sequence of SEQ ID NO: 3. Meanwhile, the gene encoding the TK protein may be a polynucleotide having a nucleotide sequence of SEQ ID NO: 4.

The term "hTERT" refers to a telomerase reverse transcriptase that synthesizes complementary DNA from the RNA template of telomerase to form an RNA-DNA hybrid form, which becomes double circular DNA and is inserted into the chromosome of the host cell. Instead of a telomere, to adhere to the chromosomal ends and continues to generate telomeres. Then, the infected host cell may be immortalized.

The term "c-Myc" refers to, a gene encoding a transcription factor located on human chromosome 8. The protein encoded by c-Myc that regulates the expression of about 15% of intracellular genes is a transcription factor, and when the transcription factor is overexpressed, cell activity and proliferation are promoted.

The host cell can be prepared by the following method:
1) primary infection of host cell with the lentiviruses comprising hTERT and/or c-Myc gene;
2) secondary infection of the primary-infected host cell with the lentiviruses comprising a tTA gene; and
3) tertiary infection of the secondary-infected host cell with the lentiviruses comprising a BDNF gene.

In the Step 1), hTERT and/or c-Myc are genes that immortalize the host cell. Genes known as immortalizing genes other than hTERT and/or c-Myc can also be used. According to one embodiment, the hTERT and c-Myc proteins may be polypeptides having an amino acid sequences of SEQ ID NO: 7 and SEQ ID NO: 5, respectively. Meanwhile, the genes coding for the hTERT and c-Myc proteins may be polynucleotides having the nucleotide sequences of SEQ ID NO: 8 and SEQ ID NO: 6, respectively.

In the Step 2), tTA is a gene capable of regulating the expression of a target protein, which means tetracycline transactivator. The Tet-off system as used herein can regulate the expression of the target protein depending on the presence or absence of tetracycline or doxycycline as described above.

According to the present invention of the mesenchymal stem cells may be characterized by expressing the BDNF protein *ex vivo* by the TRE promoter at a ratio of 100-fold or more, 110-fold or more, 120-fold or more, or 150-fold or more compared to before doxycycline treatment.

The present invention provides a pharmaceutical composition for preventing or treating a neurological disease, comprising the recombinant lentivirus or transfected host cell as an active ingredient.

Further, the present invention provides a method for preventing or treating a neurological disease, comprising the step of administering the recombinant lentivirus or the host cell transfected with the recombinant lentivirus to a subject.

In addition, the present invention provides a use of the recombinant lentivirus or the host cell transfected with the recombinant lentivirus for the prevention or treatment of a neurological disease.

The recombinant lentivirus or host cell of the present invention can exhibit a neuroprotective effect by the expression of BDNF, and thus can be used for the treatment of various central nervous disorders.

The neurological disease may be selected from the group consisting of Alzheimer's disease (AD), Parkinson's disease (PD), amyotrophic lateral sclerosis (ALS), cerebral infarction, chronic brain injury, ischemic brain disease, spinal cord injury, Huntington's disease (HD), Rett's disease (RD), stroke, multiple sclerosis, traumatic brain injury, neonatal hypoxic ischemic encephalopathy, and erectile dysfunction.

The pharmaceutical composition may further comprise a pharmaceutically acceptable carrier. The carrier may be one generally used in the preparation of medicines, which may include lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methylhydroxybenzoate, propylhydroxybenzoate, talc, magnesium stearate, mineral oil, and the like.

In addition, the pharmaceutical composition of the present invention may further comprise a pharmaceutically acceptable additive, which be selected from the group consisting of a lubricant, a wetting agent, a sweetener, a flavor, an emulsifier, a suspending agent, a preservative, and a combination thereof
The carrier may be comprised in an amount of about 1 % to about 99.99 % by weight, preferably about 90 % to about 99.99 % by weight, based on the total weight of the pharmaceutical composition of the present invention, and the pharmaceutically acceptable additive may be comprised in an amount of about 0.1 % to about 20 % by weight.

The pharmaceutical composition may be prepared in a unit dosage form by formulating with a pharmaceutically acceptable carrier and excipient according to a conventional method, or may be prepared by filling into a multi-dose container. Herein, the formulation may be in the form of a solution, a suspension, a suspension, a syrup, or an emulsion in oil or aqueous media, or in the form of an extract, powders, granules, tablets or capsules, and may additionally contain a dispersing or stabilizing agent.

Further, the present invention provides a method for preventing or treating the neurological disease as described above, comprising the step of administering a pharmaceutical composition of the present invention to a subject.

The subject may be a mammal, particularly a human. The administration route and dosage of the pharmaceutical composition may be adjusted in various ways and amounts for administration to a subject depending on the condition of a patient and the presence or absence of a side effect, and the optimal administration method and dosage may be selected by a person skilled in the art in a suitable range. In addition, the pharmaceutical composition may be administered in combination with other drugs or physiologically active substances known to have a therapeutic effects on a disease to be treated, or may be formulated in the form of combination formulation with other drugs.

When the pharmaceutical composition is administered parenterally, examples of administration include subcutaneous, ocular, intraperitoneal, intramuscular, oral, rectal, intraorbital, intracerebral, intracranial, intraspinal, intraventricular, intrathecal, intranasal, and intravenous administrations.

The above pharmaceutical composition may be administered one or four times, specifically in two divided doses. In the case of repeated administrations, they can be administered at the interval of 12 to 48 hours, 24 to 36 hours, 1 week, 2 weeks to 4 weeks, and more specifically, at the interval of 24 hours or 1 week or more. In the case of lentiviruses, the administration may be conducted in an amount of 1.0x10⁶ to 1.0x10¹² TU, specifically 1.0x10⁸ to 1.0x10¹⁰ TU for adults. On the other hand, in the case of cells, the administration may be conducted in an amount of 1.0x10⁵ to 1.0x10¹¹ cells, specifically 1.0x10⁷ to 1.0x10⁹ cells for adults. When the dose is high, the administration may be conducted several times a day.

Hereinafter, the present invention is described in detail with reference to the following Examples. However, the following Examples are intended to illustrate the present invention, and the present invention is not limited thereto.

### Example 1. Preparation of immortalized mesenchymal stem cell (MSC)

### Example 1.1. Preparation of lentiviral vector including immortalization gene

To immortalize MSCs, lentiviral vectors including immortalization genes c-Myc and/or hTERT, respectively, were prepared. In this case, a gene construct expressing a tTA protein was also inserted to use a Tet-off system.

First, a pBD lentiviral vector was constructed by substituting an EF promoter in an expression cassette of a pWPT vector (Addgene, USA) with a CMV promoter, and further linking an RSV promoter downstream thereof.

The c-Myc gene (SEQ ID NO: 6) and the thymidine kinase (TK) gene (SEQ ID NO: 4) were linked by IRES and inserted to the pBD lentiviral vector such that the expression could be regulated by the CMV promoter. The vector constructed above was named pBD-1.

Meanwhile, the hTERT gene (SEQ ID NO: 8) was inserted into the pBD lentiviral vector such that the expression could be regulated by the CMV promoter. A gene (ZeoR; SEQ ID NO: 14) having resistance to zeocin was inserted thereinto such that the expression could be regulated by the RSV promoter. The vector constructed above was named pBD-2.

Further, a tetracycline transactivator (tTA) gene (SEQ ID NO: 10) was inserted into the pBD lentiviral vector such that the expression could be regulated by the CMV promoter. A gene (PuroR; SEQ ID NO: 12) having resistance to puromycin was inserted thereinto such that the expression could be regulated by the RSV promoter. The vector constructed above was named pBD-3.

### Example 1.2. Production of lentivirus including immobilization gene

Using the lentiviral vectors constructed in Example 1.1, lentiviruses including an immortalization gene were produced by the following method.

First, Lenti-X cells (Clontech Laboratories, USA) were cultured in a 150 mm dish using a DMEM medium including 10% fetal bovine serum. Meanwhile, the lentiviral vector was extracted and quantified from DH5α *E. coli* cells using an EndoFree Plasmin Maxi Kit (Qiagen, USA).

After the cultured Lenti-X cells were washed with PBS, 3 ml of TrypLE™ Select CTS™ (Gibco, USA) was added. After the cells were left to stand at 37°C for about 5 minutes, it was confirmed that the cells were detached. The detached cells were neutralized by adding 7 ml of a DMEM medium including 10% fetal bovine serum. The neutralized cells were collected in a 50 ml tube and centrifuged at 1,500 rpm for 5 minutes. The cells were re-suspended by removing the supernatant and adding 10 ml of a DMEM culture medium including 10% fetal bovine serum.

The number of suspended cells was measured by a hemocytometer, and then 1.2×10⁷ cells were aliquoted into a 150 mm dish. When the aliquoted cells were cultured to reach about 90% confluence, the cells were transduced with a mixture of 12 µg of the lentiviral vector, 12 µg of psPAX (Addgene; gag-pol expression, packaging plasmid) and 2.4 µg of a pMD.G plasmid (Addgene; vesicular stomatitis virus G protein, VSV-G expression, envelope plasmid). Lipofectamine (Invitrogen, USA) and Plus Reagent (Invitrogen, USA) were used to transduction. Six hours after transduction, the medium was exchanged with DMEM including 10% fetal bovine serum. After the cells were cultured for an additional 48 hours, the supernatant was collected.

The obtained supernatant was mixed with a lentivirus concentration kit (Lenti-X concentrator, Clontech Laboratories, USA) and then cultured overnight at 4°C. A virus was obtained by centrifuging the supernatant under the conditions of 4°C and 4,000 rpm for 2 hours, and the virus was re-suspended in 0.5 ml of DMEM including no FBS. As a result, lentiviruses produced from pBD-1, pBD-2 and pBD-3 lentiviral vectors were prepared at concentrations of 4.0x10⁸ TU/ml, 2.0x10⁸ TU/ml and 1.2x10⁹ TU/ml, respectively.

### Example 1.3. Preparation of immortalized mesenchymal stem cells

Immortalized MSCs were prepared using the lentiviruses including the immortalization genes produced in Example 1.2.

First, bone marrow-derived MSCs were prepared by the following method. Specifically, a bone marrow aspirate was obtained from the iliac crest of a healthy donor. Coagulation was suppressed by mixing the bone marrow aspirate with 20 IU/ml heparin in a sterilized container. After the bone marrow mixture was centrifuged under the conditions of 4°C and 739 x g for 7 minutes, the supernatant was removed and was mixed with a 10-fold volume of sterilized water. A pellet of cells was obtained by centrifuging the mixture under the same conditions. The obtained pellet was suspended in a DMEM-low glucose (11885-084, Gibco, USA) medium including 20% FBS and 5 ng/ml b-FGF (100-18B, Peprotech, USA) and aliquoted into a culture flask. After the cells were cultured under the conditions of 37°C, and 5% CO₂ for 24 to 48 hours, the medium was exchanged with a new medium. The cells were subcultured with a new medium every 3 to 4 days, and after 2 weeks of culture, it was confirmed whether MSCs were present using a fluorescence cell analyzer.

The prepared MSCs were infected with the pBD-1 lentivirus produced in Example 1.2 at 100 MOI using Retronectin (Clontech Laboratories, USA). Infected cells were infected with the pBD-2 lentiviral vector at 100 MOI as described above. After infection, the cells were stabilized, and then cells infected with the pBD-2 lentivirus were selected by adding 500 µg/ml zeocin to the culture solution.

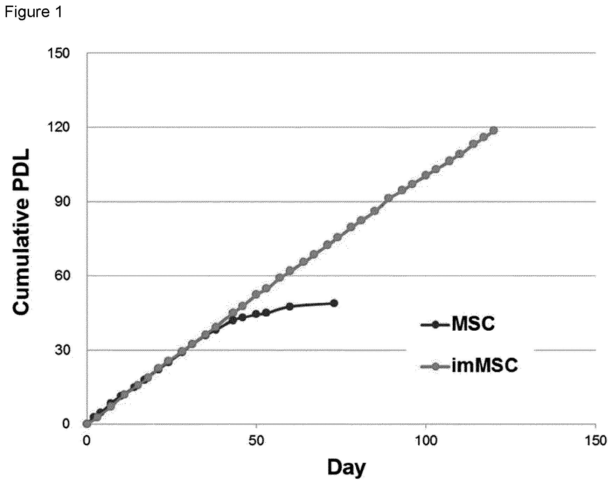

The selected cells were infected with the pBD-3 lentiviral vector at 100 MOI. After infection, cells infected with the pBD-3 lentivirus were selected by adding 1 µg/ml puromycin to a culture solution of stabilized cells. As a result, the cell proliferation rates of MSCs including the immortalization genes and those not including the immortalization genes are illustrated in FIG. 1.

As illustrated in FIG. 1, MSC cells infected with a lentivirus including immortalization genes c-Myc and hTERT maintained a high cell proliferation rate even after 120 days of culture. In contrast, the cell proliferation rate of normal MSC cells sharply decreased after 40 days of culture.

### Example 2. Construction of lentivirus including BDNF gene

### Example 2.1. Construction of lentiviral vector including BDNF gene

A BDNF gene (SEQ ID NO: 2) was inserted into the pBD lentiviral vector constructed in Example 1.1.

First, in order to confirm the effect of a promoter on BDNF expression, a lentiviral vector was prepared such that the BDNF gene would be expressed using a CMV promoter and a TRE promoter, and MSCs expressing the BDNF gene were prepared using the lentiviral vector. As a result, it was confirmed that in the case of a cell line to which the CMV promoter was applied, the MSCs expressing BDNF were morphologically changed during long-term subculture, and the expression rate of BNDF decreased as the subculture progressed.

Thus, the BNDF gene was inserted such that expression was regulated by the TRE promoter. The TRE promoter can regulate the expression of the gene linked to the promoter depending on the presence or absence of addition of doxycycline.

### Example 2.2. Production of lentivirus including BDNF gene

Using the lentivirus vector including the BDNF gene prepared in Example 2.1, a lentivirus was produced by as described in Example 1.2. The produced lentivirus was prepared at a concentration of 1.4x10¹² copies/ml.

### Example 3. Preparation of MSC transfected with lentivirus including BDNF gene

Cells expressing the BDNF gene were prepared by infecting the immortalized MSCs prepared in Example 1.3 with the lentivirus including the BDNF gene produced in Example 2.2. The transduction was performed as described in Example 1.3. After infection, the cells were stabilized, and then cells infected with pBD-4 lentivirus were selected by adding 500 µg/ml G418 to the culture media. The expression of the BDNF protein of selected cells during culture was suppressed by culture in a medium supplemented with 1 µg/ml doxycycline (631311, Clontech, USA).

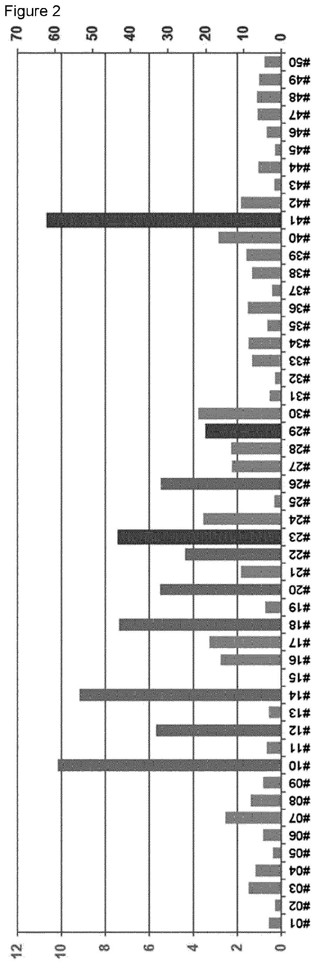

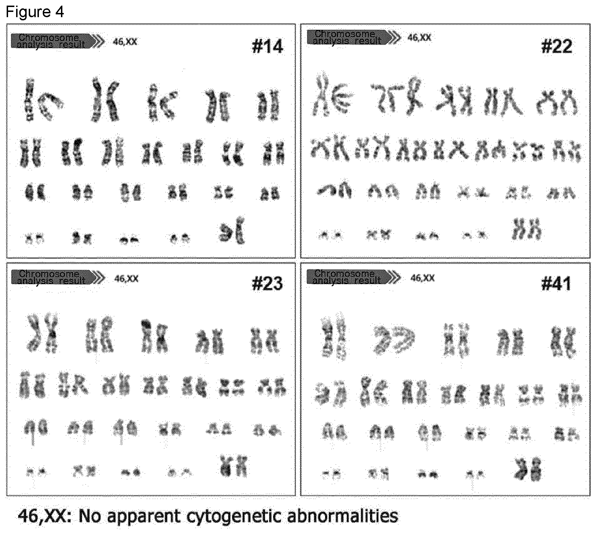

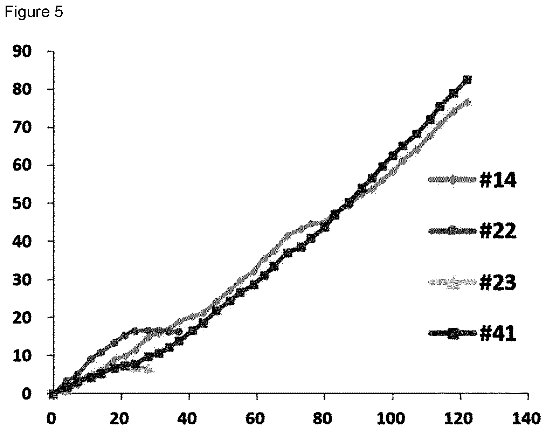

The selected cells were cultured to form colonies. Among formed clones #1 to #50 were confirmed by the presence or absence of BDNF protein expression using a human BDNF DuoSet ELISA kit (R&D Systems, DY248, USA), clones #10, #12, #14, #18, #20, #22, #23, #26, #29 and #41 expressing the BDNF protein were selected (FIG. 2). Thereafter, clones #14, #22, #23 and #41 that did not express the BDNF protein during doxycycline treatment were selected (FIG. 3). The four clones were confirmed by requesting a chromosome analysis from EONE Laboratories. All four clones showed normal chromosomal phenotype, and cell proliferation ability was confirmed for 140 days (FIGS. 4 and 5). As a result, clone #41 which showed a stable proliferation pattern and had the highest BDNF expression level was named the BM-01A cell line, and then subjected to experiments. The BM-01A cell line was deposited with Accession Number KCTC13778BP in the Korean Collection for Type Cultures of Korea Research Institute of Bioscience & Biotechnology on December 14, 2018.

### Experimental Example 1. Confirmation of surface antigen protein expression in BM-01A cell line

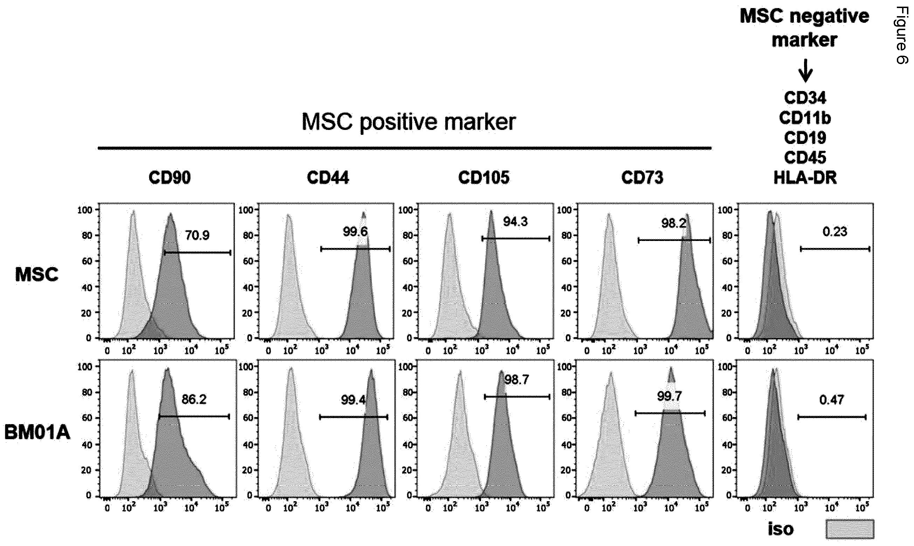

The expression of surface antigen proteins of the bone marrow-derived MSC before gene insertion and the BM-01A cell line in which the BDNF gene was inserted were analyzed using a human MSC analysis kit (Stemflow™, CatNo562245, BD). The experiment was performed according to the manual included in the kit, and the experimental results are illustrated in FIG. 6.

As illustrated in FIG. 6, it was demonstrated that even after the BM-01A cell line was subjected to gene manipulation to express BDNF, the expression of the surface antigen proteins CD90, CD44, CD105, and CD73, which are the unique properties of MSCs, was similar to that of the bone marrow-derived MSC.

### Experimental Example 2. Confirmation of proliferation rate of BM-01A cell line

The proliferation rate of the BM-01A cell line established in Example 3 was confirmed.

1.2x10⁶ to 13.0x10⁶ cells of the BM-01A cell line were inoculated into a T175 flask and cultured for 3 or 4 days, and the population doubling level (PDL) and cell viability were measured. PDL was calculated by the equation "PDL=X+3.222 (logY-logI)", where X indicated the initial PDL, I indicated the initial number of cells inoculated into a blood vessel and Y indicated the final number of cells.

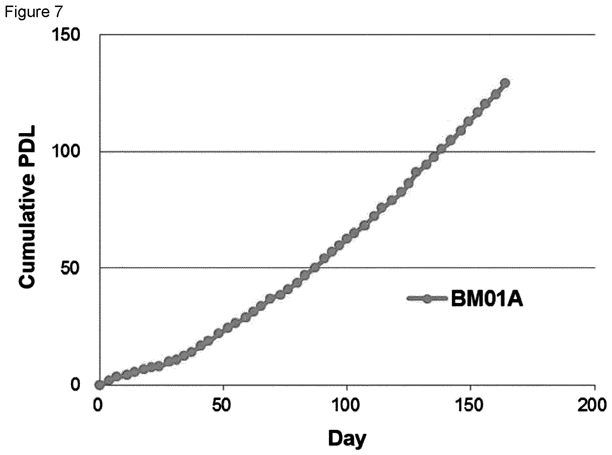

As a result, the proliferation rate of the established cell line is illustrated in FIG. 7. IN FIG. 7, the BM-01A cell line stably proliferated for up to 160 days in culture.

### Experimental Example 3. Morphological confirmation of BM-01A cell line according to subculture

In order to confirm the morphological change according to the subculture of the BM-01A cell line established in Example 3, in Experimental Example 1, the proliferation rate of the BM-01A cell line was confirmed, and the cells were photographed using microscope.

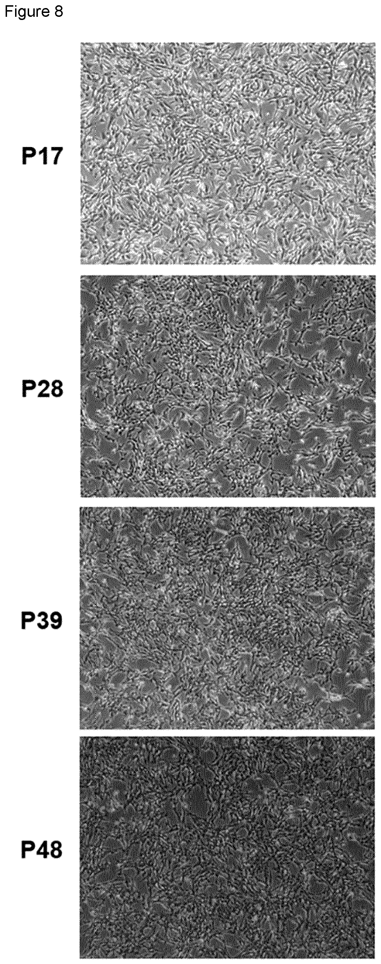

As a result, it was confirmed that BM-01A cells were not morphologically changed even after 140 days or more of long-term subculture (FIG. 8).

### Experimental Example 4. Confirmation of expression of BDNF protein in BM-01A cell line

The expression of the BDNF protein in the BM-01A cells established in Example 3 was confirmed by an ELISA analysis method. Specifically, the expression level of the BDNF protein was confirmed by a human BDNF DuoSet ELISA kit. The experiment was performed according to the manual included in the kit.

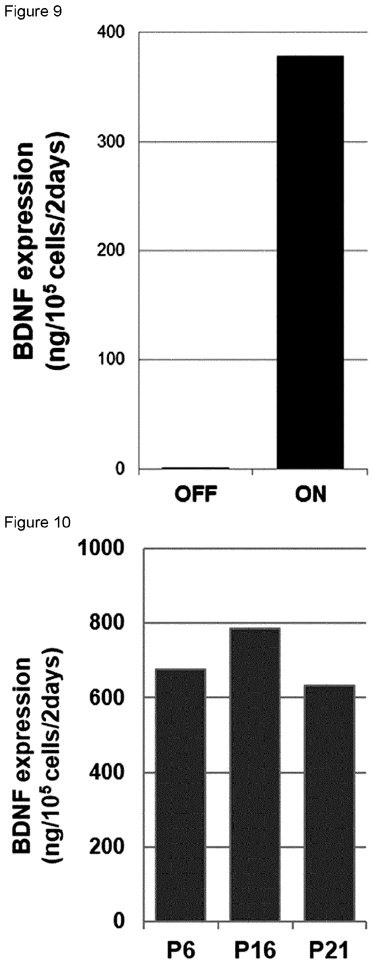

As a result of the experiment, the expression levels of the BDNF protein whose expression was induced for 48 hours from about 1×10⁵ cells in a medium with the addition or depletion of doxycycline were illustrated in the following FIG. 9. About 1.6 ng of BDNF was detected in the medium containing doxycycline, whereas about 380 ng of BDNF was detected in the medium without doxycycline.

**[Table 1]**

| Target protein | Expression level |
|---|---|
| BDNF | 380 ng/10⁵ cells |

As shown in Table 1, it was confirmed that about 380 ng/10⁵ cells of BDNF protein were expressed in the BM-01A cell line of the present invention (Table 1). In addition, the expression level of the BDNF protein at each subculture of the established BM-01A cell line was measured by a human BDNF ELISA kit. As a result, it was confirmed that the BDNF protein expression levels were constantly remained (FIG. 10).

### Experimental Example 5. Confirmation of BDNF transgene in BM-01A cell line

PCR was performed to confirm whether a gene was introduced into the BM-01A cell line prepared in Example 3. Specifically, the BM-01A cell line was transferred to a 15 ml tube containing 9 ml of PBS, and then cell down was performed at 1,500 rpm for 5 minutes. After the PBS was completely removed, the pellet was suspended in 200 µl of PBS and then transferred to a 1.5ml tube. Thereafter, gDNA was prepared using NucleoSpin® Tissue (MN, 740952.250), a mixture was prepared as shown in the following Table 2, and then PCR was performed with the steps shown in the following Table 3. In this case, 100 ng of BM-01A plasmid DNA was used as a positive control, 1 µl of purified water was used as a negative control. The BM-01A plasmid DNA can be isolated and purified by the method described in Korean Patent Application Laid-Open No. 2017-0093748.

**[Table 2]**

| | |
|---|---|
| Forward primer (SEQ ID NO: 17) (10 pmol/µl, Macrogen synthesis) | 1 µl |
| Reverse primer (SEQ ID NO: 18) (10 pmol/µl, Macrogen synthesis) | 1 µl |
| Specimen (100 ng/µl) | 1 µl |
| Purified water | 17 µl |
| Total volume | 20 µl |

**[Table 3]**

| Step | Temperature | Time | Number |
|---|---|---|---|
| 1 | 95°C | 5 minutes | 1 |
| 2 | 95°C | 30 seconds | 35 |
| | 60°C | 30 seconds | |
| | 72°C | 1 minutes | |
| 3 | 72°C | 7 minutes | 1 |
| 4 | 4°C | Unlimited | 1 |

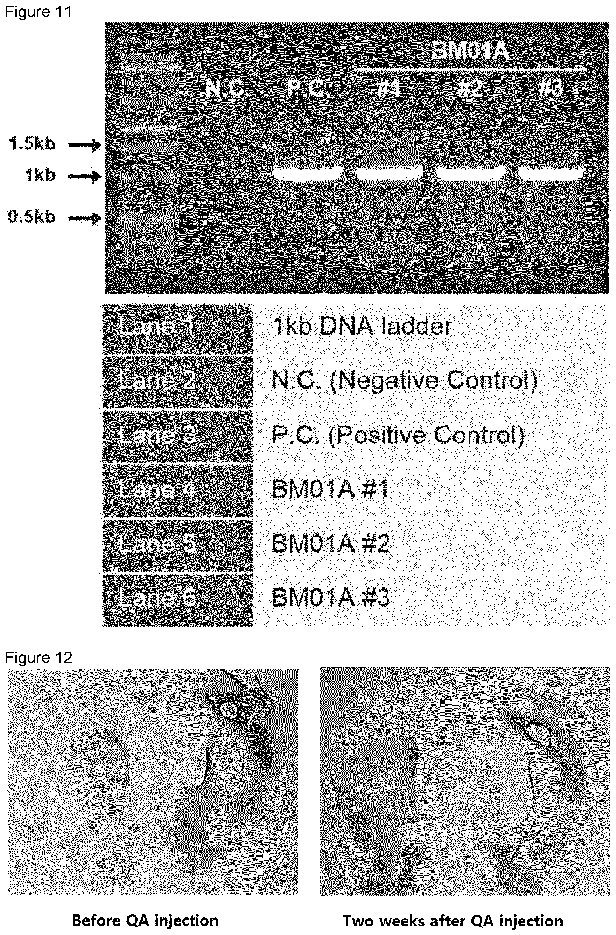

A 1% (v/v) agarose gel was put into an electrophoresis kit. 10 µl of DNA Size Marker was loaded into the first well, and from the next well, 10 µl of each of the negative control, the positive control and three BM-01A specimens were loaded. Thereafter, electrophoresis was performed at 100 V for 20 minutes, a gel photograph was taken, and the results are illustrated in FIG. 11. As illustrated in FIG. 11, all three BM-01A cell lines were confirmed to have PCR products having the same size (1.0 kb) as the positive control.

### Experimental Example 6. Confirmation of protective and therapeutic effects of BM-01A cell line on nerve cells

### Experimental Example 6.1. Production of Huntington's disease-induced rat

Acute Huntington's disease-induced rats were produced by injecting a neurotoxin, quinolinic acid (QA), into the right medial forebrain bundle (AP +1.0, ML -2.5, DV -5.0) at a concentration of 120 nmol/2 µl at a rate of 0.2 µl/min. In order to confirm the produced acute Huntington's disease animal model, immunostaining was performed using a DARPP-32 antibody (1:100, Cell Signaling) 2 weeks after QA administration, and then the volume of a lateral ventricle zone (LVZ) was measured.

As illustrated in FIG. 12, it was confirmed that the volume of the LVZ increased due to atrophy of the brain after QA administration.

### Experimental Example 6.2. Administration of BM-01A cell line

The BM-01A cell line at a concentration of 1x10⁶ cells/1 µl was administered into two medial forebrain bundle sites (AP 1.5, ML -3.0, DV -5.5 and -4.5) of the Huntington's disease-induced rat produced in Experimental Example 6.1 at a rate of 0.2 µl/min. The same dose of culture medium was administered into the control. One week after administration, the presence of the BM-01A cell line was confirmed by performing hNu immunostaining.

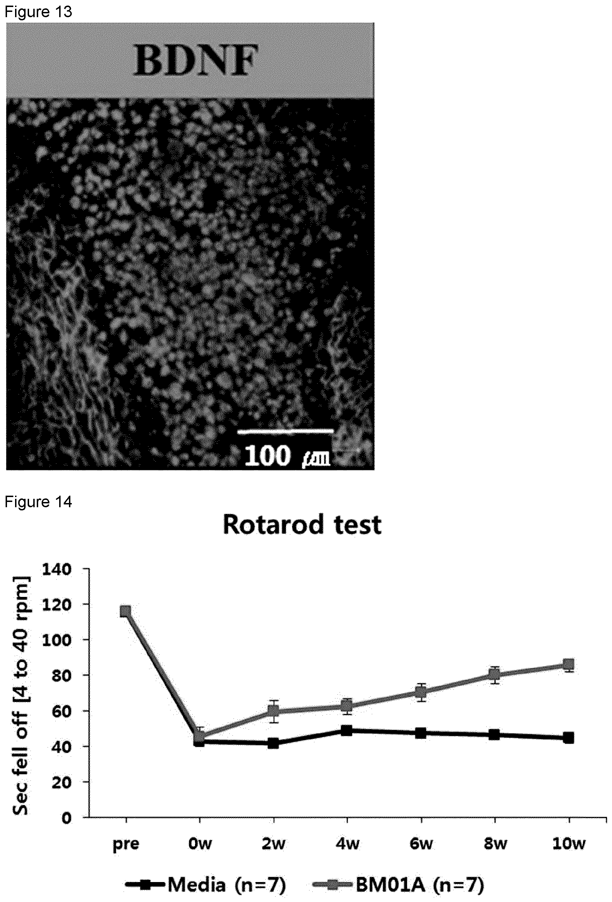

As illustrated in FIG. 13, it was confirmed that the BM-01A cell line was present in the medial forebrain bundle.

### Experimental Example 6.3. Confirmation of behavioral improvement effect

In order to confirm the behavioral improvement effect in Huntington's disease-induced rats according to the administration of the BM-01A cell line, the rats were trained for 1 week from 3 weeks before the administration of BM-01A. Behavioral experimental values of normal rats were measured 2 weeks before the administration of BM-01A and Huntington's disease was induced by injecting QA one week before the administration of BM-01A. As behavioral experiments, a Rota-rod test, a stepping test, and a staircase test were performed.

First, a Rota-rod test for confirming basic sensorimotor skills was performed. The Rota-rod test was performed in a manner of measuring the time it takes for a rat to fall from a cylinder that rotates at a gradually increasing speed. Further, the Rota-rod test was performed before QA administration, immediately after BM-01A administration, and at 2, 4, 6, 8, and 10 weeks after BM-01A administration. As a result, from 2 weeks after BM-01A administration, it took increased time for the experimental group injected with the BM-01A cell line to fall from the cylinder than for the control, and the difference between the times taken for the control and the experimental group to fall was increased as time passed (FIG. 14).

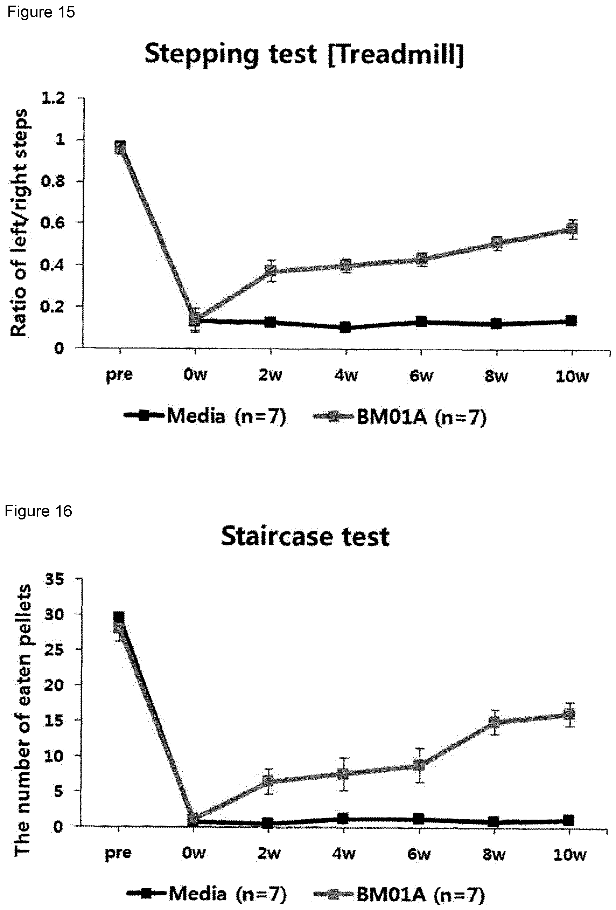

In addition, the stepping test was performed in a manner of fixing one paw of the rat and then measuring the number of times the table was touched by the another paw. The stepping test was performed 2 weeks before QA administration, immediately after BM-01A administration, and at 2, 4, 6, 8, and 10 weeks after BM-01A administration. As a result, from 2 weeks after BM-01A administration, the number of table touches in the experimental group injected with the BM-01A cell line was increased than that in the control, and the difference in the number of table touches between the control and the experimental group was increased as time passed (FIG. 15).

In addition, the staircase test was performed in a manner of setting 5 pieces of food where a staircase was installed box, and then allowing the rats to ingest the food by paw for 15 minutes, and calculating the number of pieces of ingested food by measuring the number of the remaining pieces of food. The staircase test was performed 2 weeks before QA administration, immediately after BM-01A administration, and at 2, 4, 6, 8, and 10 weeks after BM-01A administration. As a result, from 2 weeks after BM-01A administration, the number of pieces of ingested food of the experimental group administered with the BM-01A cell line was increased than that in the control, and the difference in ingested pieces of food between the control and the experimental group was increased as time passed (FIG. 16).

### Experimental Example 6.4. Confirmation of anti-inflammatory effect

The control and experimental group rats prepared in Experimental Example 6.2 were sacrificed at 10 weeks after administration. Rat brains were fixed by perfusion of 4% (v/v) paraformaldehyde. A 40 µm frozen coronal section was prepared using a cryostat (Microm, Germany).

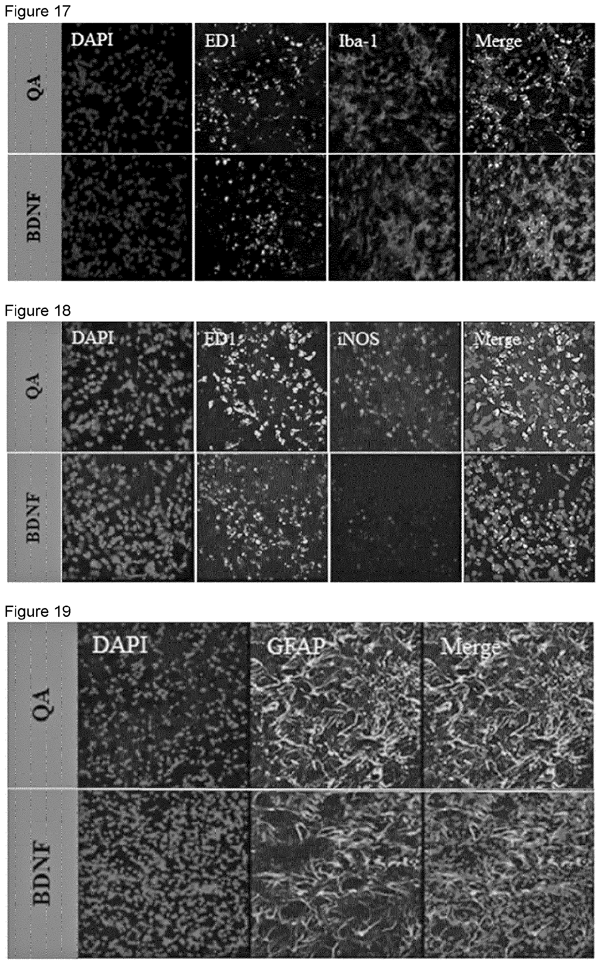

The section was stained using a rabbit-anti-mouse Iba-1 antibody and a goat-anti-rabbit IgG antibody labeled with Alexa-647 in order to stain resting state microglia and activated microglia. Thereafter, the section was stained using a rabbit-anti-mouse ED1 (CD68) antibody and a goat-anti-rabbit IgG antibody (ED1) labeled with Alexa-488 in order to confirm the activated microglia. Thereafter, the section was subsequently stained with DAPI(4,6-diamidino-2-phenylindole), and then photographs were taken using a confocal laser-scanning microscope imaging system (LSM510, Carl Zeiss, Inc.). As a result, decreases activated microglia were observed in the experimental group administered with the BM-01A cell line than in the control (FIG. 17).

Further, the section was stained using a rabbit-anti-mouse ED1 (CD68) antibody and a goat-anti-rabbit IgG antibody (ED1) labeled with Alexa-488. Thereafter, the section was stained using a rabbit-anti-mouse iNOS antibody and a goat-anti-rabbit IgG antibody labeled with Alexa-647 in order to confirm cells expressing iNOS which is an inflammatory marker in activated microglia. Subsequently, the section was stained with DAPI, and then photographs were taken using a confocal laser-scanning microscope imaging system. As a result, it was confirmed that in the experimental group administered with the BM-01A cell line, the iNOS expression level of activated microglia was decreased (FIG. 18).

### Experimental Example 6.5. Confirmation of gliosis-reducing effect

The control and experimental group rats prepared in Experimental Example 6.2 were sacrificed at 10 weeks after administration. Rat brains were fixed by perfusion of 4% (v/v) paraformaldehyde. A 40 µm frozen coronal section was prepared using a cryostat.

In the section, a glial fibrillary acidic protein (GFAP) which glial cells characteristically express was stained using a rabbit-anti-mouse GFAP antibody and a goat-anti-rabbit IgG antibody labeled with Alexa-488. Subsequently, the section was stained with DAPI, and then photographs were taken using a confocal laser-scanning microscope imaging system.

As a result, decreased glial cells were observed in the experimental group administered with the BM-01A cell line than in the control (FIG. 19).

### Experimental Example 6.6. Confirmation of neuronal protective effect

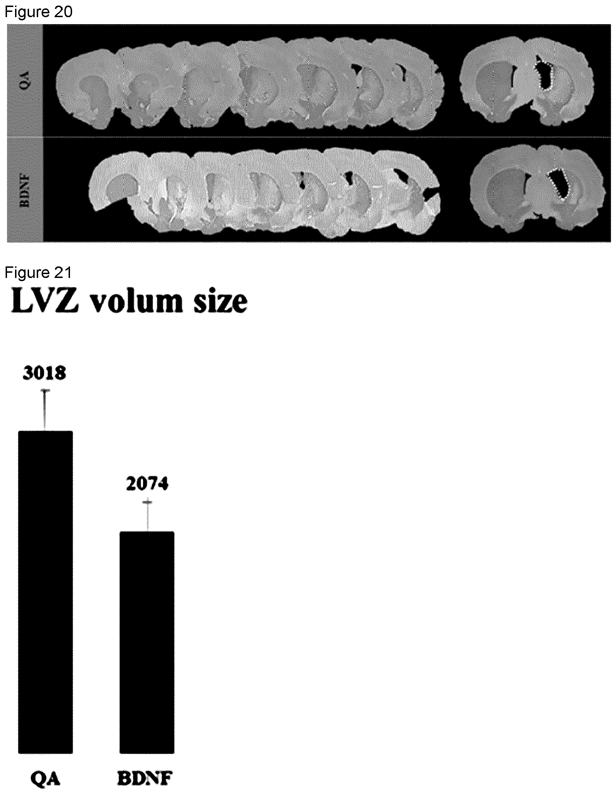

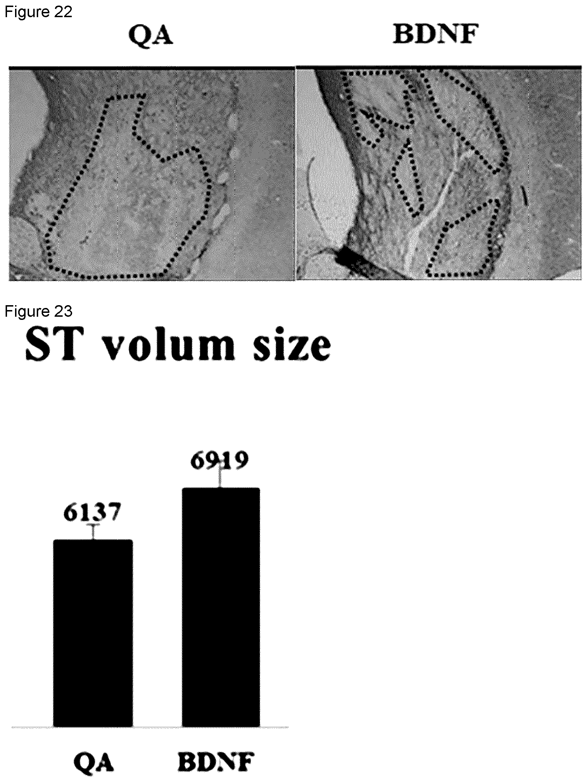

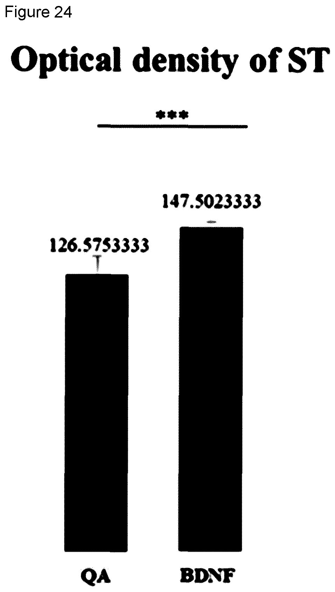

The control and experimental group rats prepared in Experimental Example 6.2 were sacrificed at 10 weeks after administration. Rat brains were fixed by perfusion of 4% (v/v) paraformaldehyde. A 40 µm frozen coronal section was prepared using a cryostat. After immunostaining using a DARPP-32 antibody, the LVZ volume, the volume of the deletion area, and the density in the striatum were measured. As a result, the LVZ volume was decreased in the experimental group administered with BM-01A (FIGS. 20 and 21). Furthermore, it was confirmed that in the experimental group administered with BM-01A, due to the administration of QA, the volume of the deletion area in the striatum was decreased and the density in the striatum was increased administration of QA (FIGS. 22 to 24).

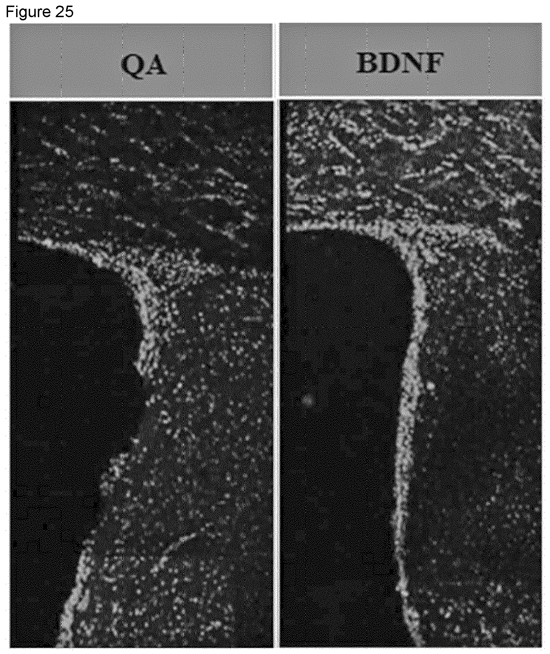

Further, in order to confirm the change in expression level of doublecortin (DCX) which is a marker protein for neurogenesis, the prepared section was stained using a rabbit-anti-mouse DCX antibody and a goat-anti-rabbit IgG antibody labeled with Alexa-647. Photographs were taken using a confocal laser-scanning microscope imaging system. As a result, it was confirmed that the DCX expression level of the experimental group administered with the BM-01A cell line was increased (FIG. 25).

### Experimental Example 7. Confirmation of protective and therapeutic effects of BM-01A cell line on nerve cells in chronic stroke-induced experimental animals

### Experimental Example 7.1. Production of chronic stroke-induced rats

In order to confirm the behavioral improvement effect in chronic stroke-induced rats according to the administration of the BM-01A cell line, rats were trained for 1 week from 5 weeks before the administration of BM-01A. Four weeks before the administration of BM-01A, stroke was induced by a middle carotid artery occlusion (MCAo) experiment in normal rats.

### Experimental Example 7.2. Confirmation of behavioral improvement effect

In order to confirm the behavioral improvement effect in stroke-induced rats according to the administration of the BM-01A cell line, rats were trained for 1 week from 3 weeks before the administration of BM-01A. Behavior experimental values of normal rats were measured by two behavioral experiments 3 weeks and 1 week before the administration of BM-01A, stroke-induced rats were selected through the behavioral experiments, BM-01A was administered intracerebrally at each dose, and then the behavioral experiment was additionally performed four times, and the behavior experimental values were measured through a total of six behavioral experiments by additionally performing the behavioral experiment four times. As the behavioral experiments, a Rota-rod test, a stepping test, and a modified neurological severity score test (mNSS) were performed.

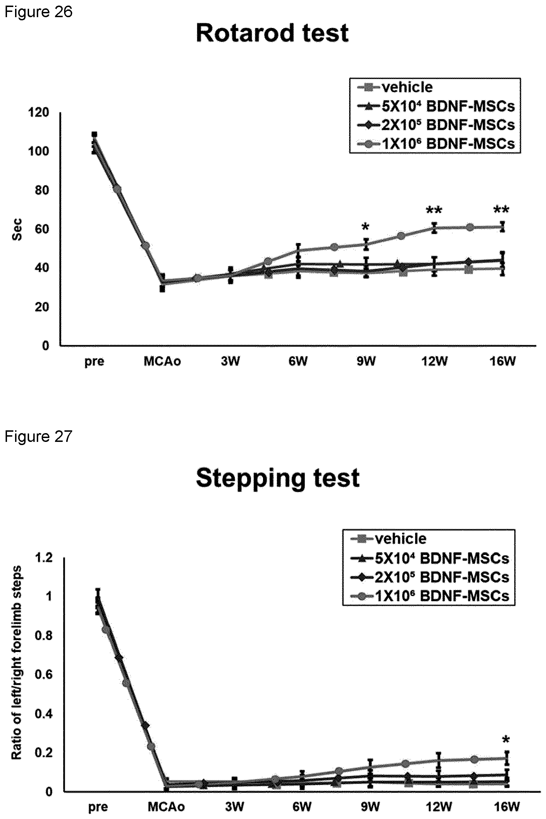

First, a Rota-rod test for confirming basic sensorimotor skills was performed. The Rota-rod test was performed in a manner of measuring the time it takes for a rat to fall from a cylinder that rotates at a gradually increasing speed. The experiment was performed 1 week before MCAo modeling, immediately and 3 weeks after MCAo modeling, and at 2, 5, 8, and 12 weeks after BM-01A administration. As a result, from 5 weeks after BM-01A administration, it took increased time for the experimental group administered with 1x10⁶ cells of the BM-01A cell line to fall from the cylinder than for the control, and the difference between the times taken for the control and the experimental group to fall was increased as time passed (FIG. 26).

In addition, the stepping test was performed in a manner of fixing one paw of the rat and then measuring the number of times the table was touched by another paw. The stepping test was performed 1 week before MCAo modeling, immediately and 3 weeks after MCAo modeling, and at 2, 5, 8, and 12 weeks after BM-01A administration. As a result, 12 weeks after BM-01A administration, the number of table touches in the experimental group administered with 1x10⁶ cells of the BM-01A cell line was increased than that in the control, and the difference in the number of table touches between the control and the experimental group was increased as time passed (FIG. 27).

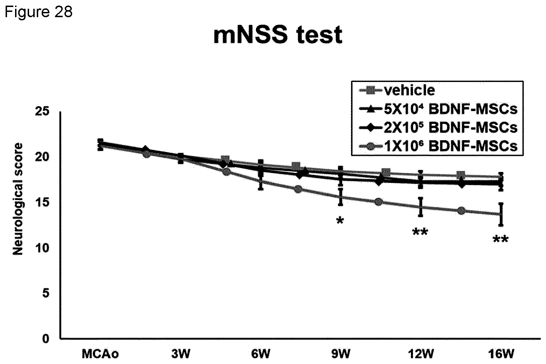

Furthermore, the modified neurological severity score test (mNSS) was scored by comprehensively evaluating motor skills, sensory function, balance maintenance ability, reflex function and the like. The experiment was performed 1 week before MCAo modeling, immediately and 3 weeks after MCAo modeling, and at 2, 5, 8, and 12 weeks after BM-01A administration (FIG. 28). As a result, from 5 weeks after BM-01A administration, the behavior index of the experimental group administered with 1x10⁶ cells of the BM-01A cell line was improved compared to that of the control, and the difference in behavior index between the control and the experimental group was increased and maintained as time passed.

### Experimental Example 7.3. Confirmation of anti-inflammatory effect

Control and experimental group rats were sacrificed at 16 weeks after administraion. Rat brains were fixed by perfusion of 4% (v/v) paraformaldehyde. A 40 µm frozen coronal section was prepared using a cryostat (Microm, Germany).

A glial fibrillary acidic protein (GFAP) which glial cells characteristically express was stained using a rabbit-anti-mouse GFAP antibody and a goat-anti-rabbit IgG antibody labeled with Alexa-488 in order to confirm the formation of glial scars in the section. Subsequently, photographs were taken using a confocal laser-scanning microscope imaging system (LSM510, Carl Zeiss, Inc.).

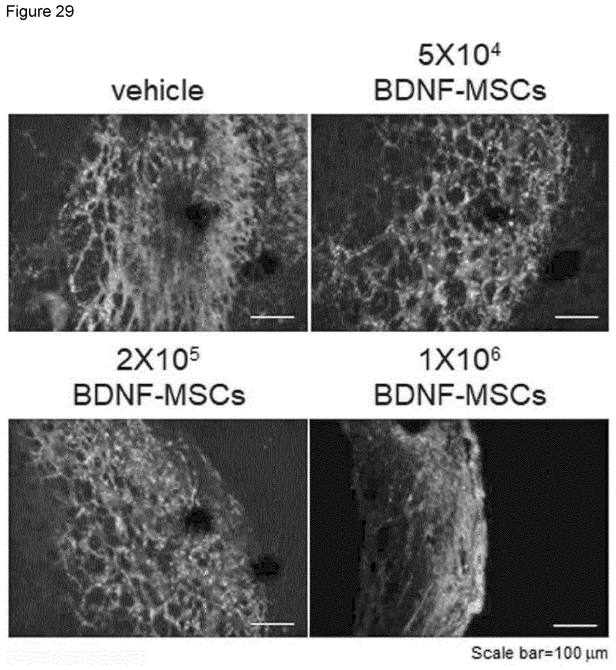

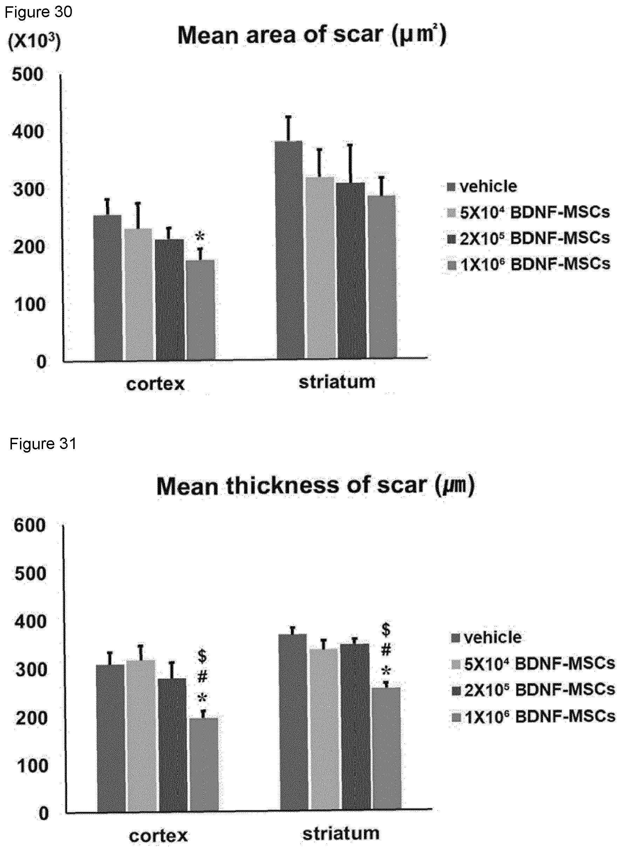

As a result, when the scar area was measured, the scar area was decreased in the cortical region in the experimental group administered 1×10⁶ cells of the BM-01A cell line compared to that in the control (FIG. 29), and in the striatal site, the scar area was decreased, but no statistical significance was observed (FIG. 30). Next, when the scar thickness was measured, it was observed that the scar thickness was decreased in the cortical and striatal sites in the experimental group administered with 1×10⁶ cells of the BM-01A cell line compared to that in the control (FIG. 31).

### Experimental Example 8. Confirmation of safety of BM-01A cell line

### Experimental Example 8.1. Confirmation of oncogenicity of in vitro BM-01A cell line

It was confirmed whether a colony was formed by anchorage-independent growth of a bone marrow-derived MSC, a BM-01A cell line, a positive control (HeLa), and a negative control (NIH3T3) in soft agar gels using a CytoSelect™ 96-well Cell Transformation Assay, and tumor formation due to cell transformation was quantified by staining with a MTS solution and measuring absorbance.

As a result, colonies were formed by cell transformation in the positive control, and colonies were not formed in the negative control, the bone marrow-derived MSC, and the BM-01A cell line. It could be confirmed that there was no transformation ability during cell culture and there was no *in vitro* oncogenicity (FIG. 32).

### Experimental Example 8.2. Confirmation of safety of in vivo BM-01A cell line

In order to confirm the *in vivo* safety of BM-01A, it was confirmed whether the BM-01A cell line survived by sacrificing experimental rats in Experimental Example 6.2 at 10 weeks after administration. Specifically, control and experimental group rats were sacrificed in Experimental Example 6.2 at 10 weeks after administration. Rat brains were fixed by perfusion of 4% (v/v) paraformaldehyde. A 40 µm frozen coronal section was prepared using a cryostat. Immunostaining was performed using an anti-human nuclei antibody and an apototic marker anti-Caspase3 antibody. Photographs were taken using a confocal laser-scanning microscope imaging system.

As a result, the human nuclei were present in an apoptotic form, and the Caspase 3 was co-stained. That is, it was demonstrated that all of the BM-01A cell line was dead, and it was confirmed through this experiment that safety was excellent because the BM-01A cell line did not abnormally differentiate nor proliferate even *in vivo* (FIG. 33).

### [Accession Number]

Name of Depositary Institution: Korea Research Institute of Bioscience & Biotechnology
Accession Number: KCTC13778BP
Date of deposit: December 14, 2018

Applicant's or agent's
file reference PCT 5040170 | International application No.

INDICATIONS RELATING TO DEPOSITED MICROORGANISM
OR OTHER BIOLOGICAL MATERIAL

(PCT Rule 13bis)

A. The indications made below relate to the deposited microorganism or other biological material referred to in the description
on page 13 , line 17 .

B. IDENTIFICATION OF DEPOSIT | Further deposits are identified on an additional sheet [ ]

Name of depositary institution
Korean collection for type cultures

Address of depositary institution (including postal code and country)
(56212) 181 Ipsin-gil, Jeongeup-si, Jeollabuk-do, Republic of Korea, Korea Research Institute of Bioscience
& Biotechnology (KRIBB)

Date of deposit | Accession Number
December 14, 2018 | KCTC 13778BP

C. ADDITIONAL INDICATIONS (leave blank if not applicable) | This information is continued on an additional sheet [ ]

D. DESIGNATED STATES FOR WHICH INDICATIONS ARE MADE (if the indications are not for all designated States)

E. SEPARATE FURNISHING OF INDICATIONS (leave blank if not applicable)

The indications listed below will be submitted to the International Bureau later (specify the general nature of the indications e.g., "Accession
Number of Deposit")

For receiving Office use only | For International Bureau use only
[ ] This sheet was received with the international application | [ ] This sheet was received by the International Bureau on:
Authorized officer | Authorized officer

Form PCT/RO/134 (July1998; reprint January 2004)

## Claims

1. A recombinant lentiviral vector comprising a gene encoding a brain-derived neurotrophic factor (BDNF) protein.

2. The recombinant lentiviral vector of claim 1, wherein the BDNF protein is a polypeptide having an amino acid sequence of SEQ ID NO: 1.

3. The recombinant lentiviral vector of claim 1, wherein the gene encoding the BDNF protein has a nucleotide sequence of SEQ ID NO: 2.

4. The recombinant lentiviral vector of claim 1, wherein the vector comprises promoter.

5. The recombinant lentiviral vector of claim 4, wherein the promoter is a cytomegalovirus (CMV), respiratory syncytial virus (RSV), human elongation factor-1 alpha (EF-1α) or tetracycline response element (TRE) promoter.

6. A recombinant lentivirus comprising a gene encoding a brain-derived neurotrophic factor (BDNF) protein.

7. The recombinant lentivirus of claim 6, wherein the lentivirus is obtained through the steps of:
transforming a host cell with the lentiviral vector of claim 1, a packaging plasmid, and an envelope plasmid; and
isolating the lentivirus from the transformed host cell.

8. A host cell transfected with the recombinant lentivirus of claim 6.

9. The transfected host cell of claim 8, wherein the host cell is an immortalized mesenchymal stem cell.

10. The transfected host cell of claim 8, wherein the host cell has an hTERT and/or c-Myc gene introduced therein.

11. A pharmaceutical composition for preventing or treating a neurological disease, comprising the recombinant lentivirus of claim 6 as an active ingredient.

12. The pharmaceutical composition of claim 11, wherein the neurological disease is selected from the group consisting of Alzheimer's disease (AD), Parkinson's disease (PD), amyotrophic lateral sclerosis (ALS), cerebral infarction, chronic brain injury, spinal cord injury, Huntington's disease (HD), Rett's disease (RD), ischemic brain disease, stroke and traumatic brain injury, and multiple sclerosis.

13. A pharmaceutical composition for preventing or treating a neurological disease, comprising the transfected host cell of claim 8 as an active ingredient.

14. The pharmaceutical composition of claim 13, wherein the neurological disease is selected from the group consisting of Alzheimer's disease (AD), Parkinson's disease (PD), amyotrophic lateral sclerosis (ALS), cerebral infarction, chronic brain injury, spinal cord injury, Huntington's disease (HD), Rett's disease (RD), ischemic brain disease, stroke and traumatic brain injury, and multiple sclerosis.

15. Use of the recombinant lentivirus of claim 6 or the host cell transfected with the recombinant lentivirus for prevention or treatment of a neurological disease.

16. Method for preventing or treating a neurological disease, the method comprising: administering the recombinant lentivirus of claim 6 or the host cell transfected with the recombinant lentivirus to an individual.
